# EUROPEAN PATENT APPLICATION

(11) **EP 4 230 701 A1**
(43) Date of publication of application: **23.08.2023**
(21) Application number: 22216793.4
(22) Date of filing: 27.12.2022
(51) Int. Cl.: C09B 23/14, C09K 11/06, G01N 33/533

(54) **4-STYRYL COUMARINS DERIVATIVES AS FLUORESCENT LABELS FOR BIOMOLECULES**

(30) Priority: 18.02.2022 PT 2022117795
(71) Applicant: Universidade de Évora, 7000-803 Évora (PT)
(72) Inventor: FIALHO CAEIRO CALDEIRA, Ana Teresa, 7000-651 Évora (PT); DEOMÉTRIO RODRIGUES LOURENÇO PEREIRA, António Manuel, 7000-890 Évora (PT); CACHOLA EUSTÁQUIO, Raquel, 7090-027 ALCÁÇOVAS (PT); CRISTÓVÃO PRATES RAMALHO, Joâo Paulo, 7005-351 Évora (PT); ESTEVÃO GRANDE CANDEIAS, António José, 7000-651 Évora (PT)
(74) Representative: Neves, Ana

(57) **Abstract**

Important scientific areas such as cellular biology, medicine, pharmacy, environmental sciences are dependent on very sensitive analytical techniques to track and detect biomolecules. The present application discloses a simple, low cost and effective synthetic strategy to produce new red-shifted 4-styrylcoumarin derivatives of Formulas I, II, III and IV as promising inexpensive fluorescent labels for biomolecules. The extension of the delocalized π-electron system results in bathochromic shifts in these new coumarin derivatives, which also present large Stokes shifts. In addition, density functional theory and time dependent density functional theory calculations helped to rationalize photophysical properties observed by the experimental results.

## Description

### Technical field

The present invention relates to compounds of Formula I, II, III and IV that are 4-styrylcoumarin derivatives suitable for use as fluorescent labels for biomolecules.

### Background art

Cellular biology, medicine, pharmacy and environmental sciences, require highly sensitive analytical techniques to track and detect nucleic acids, oligonucleotides, antibodies, amino acids, proteins, lipids, carbohydrates, and other biomolecules [1-6]. Most available techniques generally require labelling with a sensor, as fluorescent labels [7,8] electrochemical sensors [9], photochromic compounds [10], photo switchable biomaterials [11], colorimetric biosensors [12], radioactive tracers [13] and isotope markers [14]. Of all sensitive analytical techniques, fluorescent labelling, taking into account the high sensitivity of the fluorescence technique and their non-destructive nature, present numerous advantages as they allow the use of small sample quantities as well as the respective fluorescent labels [15] . The availability and the development of new fluorophores are now enabling previously impossible studies of cellular processes and the detection of specific components of complex biomolecular assemblies with selectivity and exquisite sensitivity, in vitro and in vivo, as well the analysis of their interactions [16-18]. Fluorescent labels offer many advantages as they are highly sensitive, even at very low concentrations, and can form covalent linkages with the sample to be analysed producing stable bioconjugates [19]. They should be chemically stable and small in size, with insignificant interference on the biological functions and structure of the unlabelled biomolecules, producing high fluorescence quantum yields bioconjugates. The amine-reactive fluorescent labels, since amino groups are either abundant or easily introduced into biomolecules, are the most frequently used to prepare numerous bioconjugates for direct or indirect immunochemistry, fluorescence in situ hybridization (FISH), histochemistry, cell tracing, receptor binding and other biological applications [20-22]. In this context, due to the high cost of the available commercial fluorescent labels, coumarin derivatives can be a solution to develop low-cost new fluorophores with absorption and emission at long wavelengths, combined with large Stokes shifts. Coumarins, beyond their very significant pharmacological activity [23,24], are interesting alternatives for applications in research focused on emission layers in organic light-emitting diodes [25], solar cells dyes [26], nonlinear optical chromophores [27], fluorescent brighteners [28], fluorescent whiteners [29], fluorescent labels for physiological measurement [30], and more recently in labelling [31] and in caging [32]. Developments of the last decades evidenced that the substitution pattern and the nature of the substituents in the coumarin rings involving donor-π bridge-acceptor structures (D-π-A) promote the delocalization of the conjugated π electron system, producing derivatives with extraordinary spectroscopic and photophysical properties [26,33,34]. Nevertheless, shifting the absorption maxima to the red region, in the particular case of the coumarin derivatives, is still a big challenge. The diethylamino electron-donating group (EDG) at position 7 creates an evident push-pull effect with the electron-withdrawing lactone moiety, which induces larger red shifts in the absorption spectrum than other EDG substituents such as alkoxy groups [35] . In this work, we developed a simple, low cost and effective synthetic strategy to produce new red-shifted 4-styrylcoumarin derivatives using 7-diethylamino-4methylcoumarin (1) as a starting material, to produce fluorescent labels for biomolecules. Additionally, Density Functional Theory (DFT) and Time Dependent Density Functional Theory (TDDFT) calculations has been conducted in some of the coumarin derivatives, in order to gain deeper insight into their electronic and spectroscopic properties.

### General description

The present invention relates to compounds of Formula I, II, III and IV that are 4-styrylcoumarin derivatives suitable for use as fluorescent labels for biomolecules.

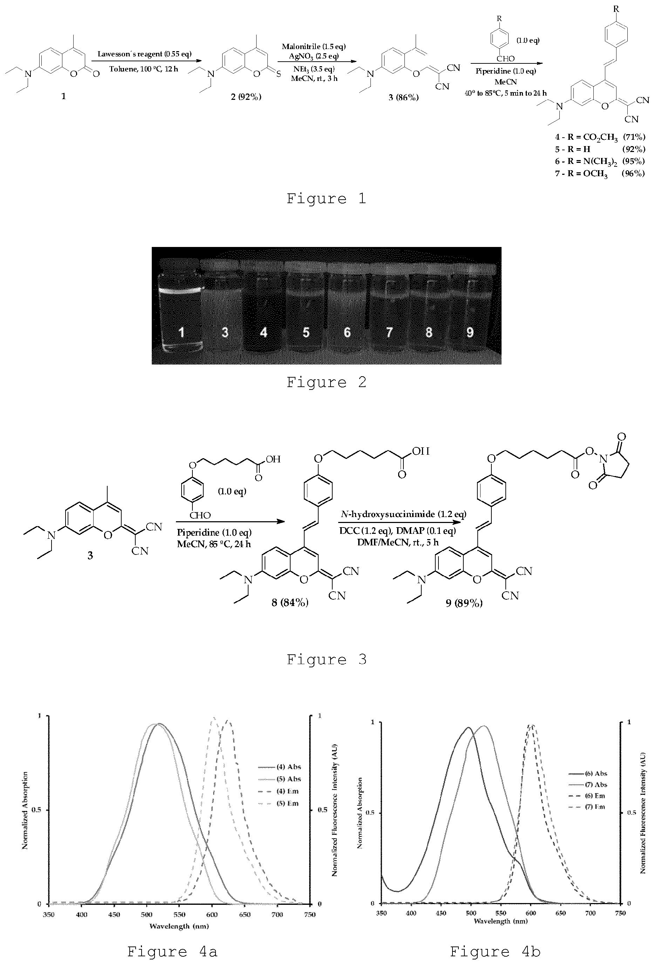

Formula I was obtained from the synthesis strategy shown in Figures 1 and 3. 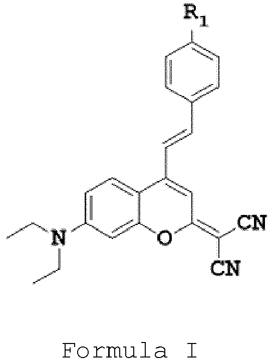 wherein
R₁ is selected from CO₂CH₃, H, N(CH₃)₂, OCH₃, 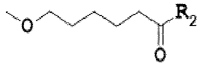 or 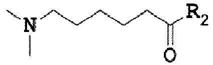
and R₂ is selected from OH,

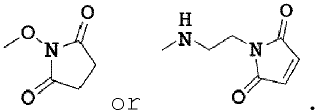

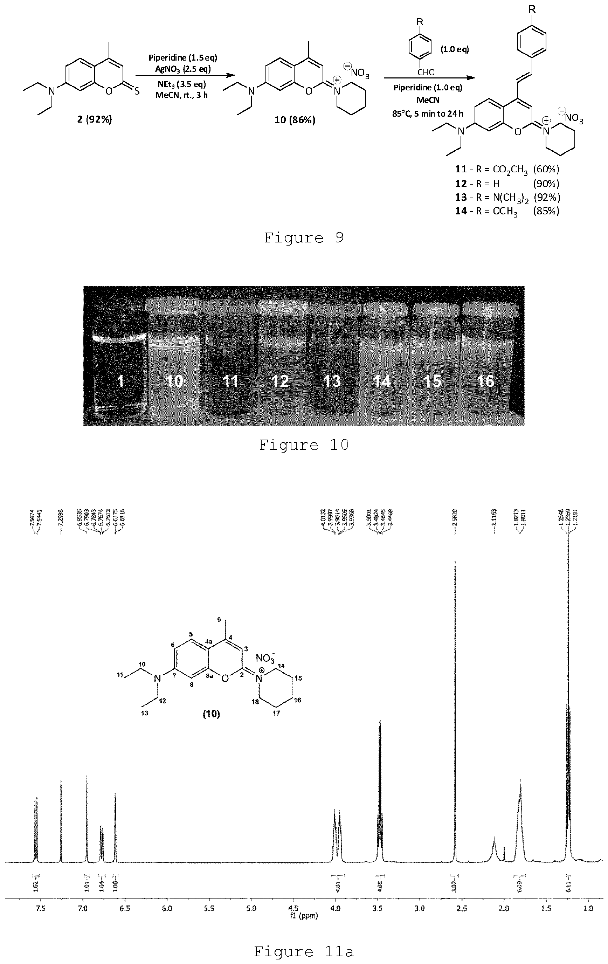

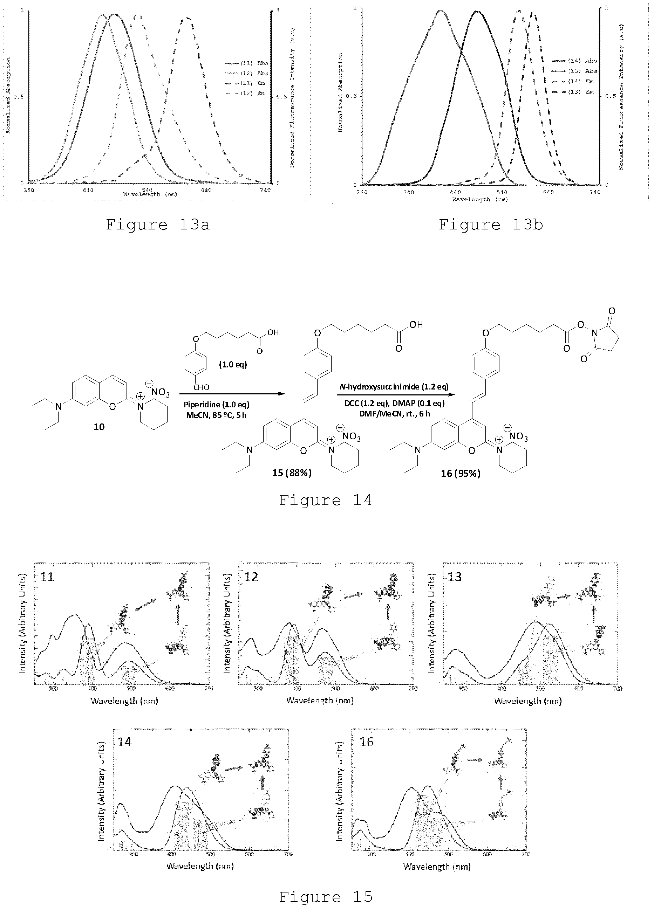

Formula II was obtained from the synthesis strategy shown in Figures 9 and 14. 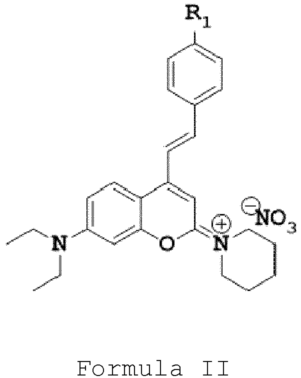 wherein
R₁ is selected from CO₂CH₃, H, N(CH₃)₂, OCH₃, 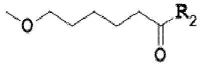 or 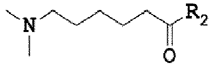
and R₂ is selected from OH,

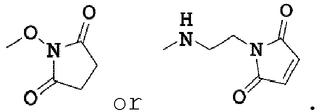

As an example, the present application discloses 4-styrylcoumarin derivative compounds of Formula I: (4), (5), (6), (7), (8), (9), and of Formula II: (11), (12), (13), (14), (15), and (16).

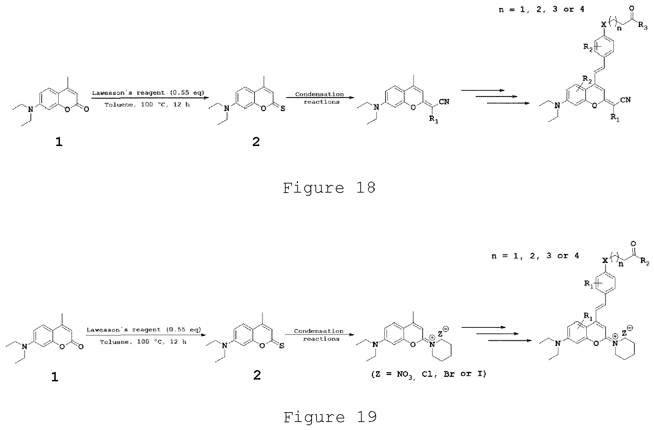

Formula III was obtained from the synthesis strategy shown in Figure 18. 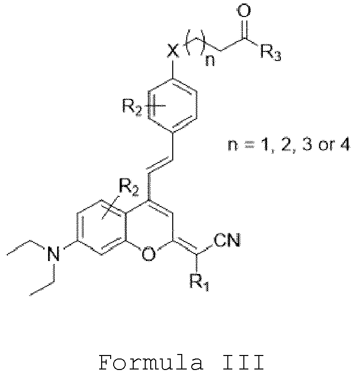 wherein
X is selected from NCH₃, O, N or S;
R₁ is selected from:
-COOCH₃, -COC₆H₄COOCH₃, -C₆H₄COOCH₃, -CN or 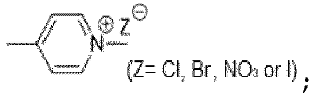
R₂ is selected from H or SO₃Na;
R₃ is selected from:
OH,

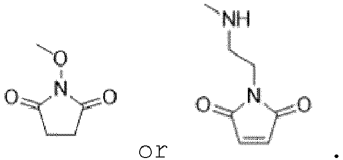

Formula IV was obtained from the synthesis strategy shown in Figure 19. 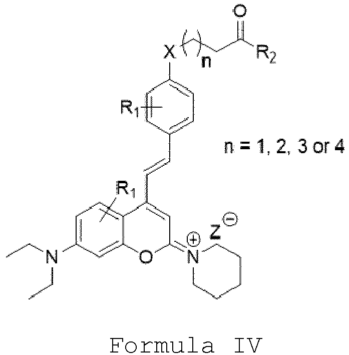 wherein
X is selected from NCH₃, O, N or S;
Z is selected from Cl, Br, NO₃ or I;
R₁ is selected from H or SO₃Na;
R₂ is selected from:
   OH,

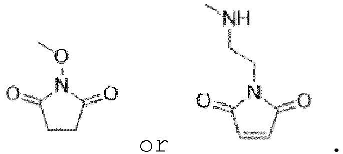

As an example, the present application discloses 4-styrylcoumarin derivative compounds of Formula III: (8) and (9), and of Formula IV: (15) and (16).

Formulas I and II derivate from the general Formulas III and IV, respectively.

### Brief description of drawings

Figure 1 shows the synthesis of compounds of Formula I.
Figure 2 shows the compounds 1, 3-9 in MeCN at 365 nm.
Figure 3 shows the synthesis of an amine-reactive fluorescent label 8 and 9 for biomolecules.
Figures 4a and 4b show absorbance and emission spectra of the new 4-styrylcoumarin derivatives (4 to 7).
Figure 5 shows optimized structures of the compounds in the ground state S0 and in the first excited state S1.
Figure 6 shows calculated molecular orbitals involved in the lowest energy S0 → S1 and S0 → S2 transitions and respective wavenumber and oscillator strengths.
Figure 7a shows the 1H-NMR spectrum of 4 (CDCl3, 400 MHz).
Figure 7b shows the 13C-NMR spectrum of 4 (CDCl3, 100 MHz).
Figure 7c shows the 1H-NMR spectrum of 5 (CDCl3, 400 MHz).
Figure 7d shows the 13C-NMR spectrum of 5 (CDCl3, 100 MHz).
Figure 7e shows the 1H-NMR spectrum of 6 (CDCl3, 400 MHz).
Figure 7f shows the 13C-NMR spectrum of 6 (CDCl3, 100 MHz).
Figure 7g shows the 1H-NMR spectrum of 7 (CDCl3, 400 MHz).
Figure 7h shows the 13C-NMR spectrum of 7 (CDCl3, 100 MHz).
Figure 7i shows the 1H-NMR spectrum of 8 (CDCl3, 400 MHz).
Figure 7j shows the 13C-NMR spectrum of 8 (CDCl3, 100 MHz).
Figure 7k shows the 1H-NMR spectrum of 9 (CDCl3, 400 MHz).
Figure 7l shows the 13C-NMR spectrum of 9 (CDCl3, 100 MHz).
Figure 8a shows the Mass spectrum of 4.
Figure 8b shows the Mass spectrum of 5.
Figure 8c shows the Mass spectrum of 6.
Figure 8d shows the Mass spectrum of 7.
Figure 8e shows the Mass spectrum of 8.
Figure 8f shows the Mass spectrum of 9.
Figure 9 shows the synthetic route to compounds of Formula II.
Figure 10 shows images of compounds (1, 10-16) in MeCN at 365 nm.
Figure 11a shows the 1H-NMR spectrum of 10 (CDCl3, 400 MHz).
Figure 11b shows the 13C-NMR spectrum of 10 (CDCl3, 100 MHz).
Figure 11c shows the 1H-NMR spectrum of 11 (CDCl3, 400 MHz).
Figure 11d shows the 13C-NMR spectrum of 11 (CDCl3, 100 MHz).
Figure 11e shows the 1H-NMR spectrum of 12 (CDCl3, 400 MHz).
Figure 11f shows the 13C-NMR spectrum of 12 (CDCl3, 100 MHz).
Figure 11g shows the 1H-NMR spectrum of 13 (CDCl3, 400 MHz).
Figure 11h shows the 13C-NMR spectrum of 13 (CDCl3, 100 MHz).
Figure 11i shows the 1H-NMR spectrum of 14 (CDCl3, 400 MHz).
Figure 11j shows the 13C-NMR spectrum of 14 (CDCl3, 100 MHz).
Figure 11k shows the 1H-NMR spectrum of 15 (CD3OD, 400 MHz).
Figure 11l shows the 13C-NMR spectrum of 15 (CD3OD, 100 MHz).
Figure 11m shows the 1H-NMR spectrum of 16 (CDCl3, 400 MHz).
Figure 11n shows the 13C-NMR spectrum of 16 (CDCl3, 100 MHz).
Figure 12a shows the Mass spectrum of 10.
Figure 12b shows the Mass spectrum of 11.
Figure 12c shows the Mass spectrum of 12.
Figure 12d shows the Mass spectrum of 13.
Figure 12e shows the Mass spectrum of 14.
Figure 12f shows the Mass spectrum of 15.
Figure 12g shows the Mass spectrum of 16.
Figure 13a and 13b shows absorbance and emission spectra of the compounds 11 to 14.
Figure 14 shows the synthesis of an amine-reactive fluorescent label for biomolecules of Formula II.
Figure 15 shows experimental spectra compared with the calculate spectra and contour plots of the molecular orbitals involved in the two lowest energy excitations.
Figure 16 shows the contour plots of the electron density difference Δρ(r) upon the lowest energy excitations for the studied compounds. Blue colour indicates an increase of electron density upon the transition while pink represents a depletion of electron density.
Figure 17 shows optimized structures of the compounds in the ground S0 and excited S1 states.
Figure 18 shows the synthesis of compounds of formula III.
Figure 19 shows the synthesis of compounds of formula IV.

### Detailed description of embodiments

With the objective to extend the delocalization of the π-electron system, new 4-styrylcoumarin derivatives (Formula I) were synthesized, with absorption and emission at long wavelengths, combined with large Stokes shifts, using a simple, low cost and efficient synthetic strategy. The results obtained from the UV/Vis spectra allow to conclude that the 2-(7-(diethylamino)-4-methyl-2H-chromen-2-ylidene)malononitrile (3) is a useful intermediate to the synthesis of promising new fluorescent labels for biomolecules. DFT and TDDFT calculations helped rationalize the observed photophysical properties, particularly the large Stokes shifts, ascribed to a significant structure relaxation found in the molecules excited states. The development of further red-shifted coumarin fluorescent labels for biomolecules with improved features is currently in progress in our laboratory and the results will be reported briefly.

New 2-piperidinium-4-styrylcoumarin derivatives (Formula II) were also synthesized, with large Stokes shifts and high fluorescence quantum yields, using an efficient and low-cost synthetic strategy. The results obtained from the UV/Vis spectra allow to conclude that the 1-(7-(diethylamino)-4-methyl-2H-chromen-2-ylidene)piperidin-1-ium nitrate (10) is a useful intermediate to the synthesis of promising new fluorescent labels for biomolecules. The conducted DFT and TD-DFT calculations found significant geometric differences in the ground and excited states that helped to rationalize the emission process.

New 4-styrylcoumarin derivatives (Formula III) were also obtained, using 2-(7-(diethylamino)-4-methyl-2H-chromen-2-ylidene)malono-nitrile (3) as a useful intermediate to the synthesis of promising new fluorescent labels for biomolecules with absorption and emission at long wavelengths, combined with large Stokes shifts, through a simple, low cost and efficient synthetic strategy.

New 4-styrylcoumarin derivatives (Formula IV) were also obtained, using 1-(7-(diethylamino)-4-methyl-2H-chromen-2-ylidene)piperidin-1-ium nitrate (10) as a useful intermediate to the synthesis of promising new fluorescent labels for biomolecules with absorption and emission at long wavelengths, combined with large Stokes shifts, through a simple, low cost and efficient synthetic strategy.

### Example 1

### Synthesis

The main synthetic strategy to obtain 4-styrylcoumarin derivatives (Formula I and III) was based on the high acidity of the methyl protons present at position 4 in 2-(7-(diethylamino)-4-methyl-2Hchromen-2-ylidene)malononitrile (3), that enable aldol condensation reactions. The mentioned dicyanomethylene-coumarinylmethyl derivative, presenting a higher bathochromic shift (more than 100 nm) when compared with its precursor (2), was obtained by the incorporation of two cyano groups in position 2 after the thionation of (1) [35,36]. It was reasoned that the incorporation of one 4-styryl group containing electron-donating groups (EDGs) or electron-withdrawing groups (EWGs) at para position, could increase the π delocalization and the push-pull character of the chromophore. These modifications can promote large bathochromic shifts in the absorption and emission bands, as well as improve other photophysical properties. According to the above, it is herein described the design, synthesis, and spectroscopic characterization of a small library of 4-styrylcoumarin derivatives to explore the effect of EWGs and EDGs on the photophysical properties of the new chromophores. Moreover, one of the best candidates considering the binomial cost/photophysical properties, has been functionalized through a reactive succinimidyl ester as an effective fluorescent label for biomolecules. The synthetic routes followed for the preparation of the novel 4-styrylcoumarin derivatives are shown in Figure 1. The stereoselective and highly efficient aldol condensation reaction between the intermediate (3) and the aromatic aldehydes (benzaldehyde, 4-(dimethylamino)benzaldehyde, 4-methoxybenzaldehyde and methyl 4-formylbenzoate), afforded the 4-styrylcoumarin derivatives (4 to 7), in very good to high yields.

Unexpected, despite needing stronger reaction conditions, aldehydes containing electrondonating groups, gave better yields than the one with electron-withdrawing group at para position, which may be due to the superior stabilization of the intermediate alcohol, by the first ones, in this kind of molecules. The aforementioned new π-extended coumarins (Figure 2) are easily isolated after silica column chromatography. All spectral data were consistent with the proposed structures (SI).

Considering the good photophysical properties induced by the methoxy group in the 4-styrylcoumarin derivative (3) this compound was selected to synthesize the amine-reactive fluorescent label (9) (Figure 3). The referred fluorescent label can be obtained through five effective and linear synthetic steps from cheap commercially available precursors and as it will be shown ahead, its photophysical properties are very similar to the derivative (7). The aldol condensation of (3) with the 6-(4-formylphenoxy)hexanoic acid afforded compound (8) which was further reacted with N-hydroxysuccinimide to attain the fluorescent label (9) (Figure 3).

### Photophysical properties

The photophysical properties of the synthesized coumarin derivatives were studied, and their absorption and emission properties, as well as fluorescence quantum yields, are summarized in Table 1.

**Table 1. Spectroscopic properties of 7-diethylamino-4-methylcoumarin derivatives.**

| Compound | λabs^{a} (nm) | λemb (nm) | Stokes shift (nm, cm⁻¹) | ε^{c} (cm⁻¹M⁻¹) | ΦFd |
|---|---|---|---|---|---|
| 1 | 371 | 434 | 63, 3913 | 22910 | 0.73 |
| 3 | 477 | 519 | 42, 1697 | 38000 | 0.05 |
| 4 | 520 | 620 | 100, 3102 | 19000 | 0.04 |
| 5 | 516 | 603 | 87, 2796 | 17000 | 0.16 |
| 6 | 496 | 597 | 101, 3411 | 34000 | 0.95 |
| 7 | 522 | 602 | 80, 2546 | 24000 | 0.20 |
| 8 | 519 | 596 | 77, 2489 | 24000 | 0.28 |
| 9 | 523 | 597 | 74, 2370 | 24000 | 0.29 |

| | | | | | |
|---|---|---|---|---|---|
| ^{a} Absorption maxima in acetonitrile. ^{b} Emission maxima in acetonitrile. ^{c} Molar extinction coefficient at longest wavelength transition. ^{d} Fluorescence quantum yield in ethanol, determined using either 7-diethylamino-4-methylcoumarin (Φ_{F} = 0.73 in ethanol) as a standard. | | | | | |

The absorption and emission spectra of the new 4-styrylcoumarin derivatives (4 to 7) are displayed in Figure 4. All referred coumarin derivatives exhibit absorption and emission maxima at longer wavelengths, when compared to intermediate, dicyanomethylenecoumarinmethyl derivative (3), due to the intramolecular charge transfer (ICT) effect by conjugation of both the electron-donating NEt2 group and the styryl groups in position 4 with the electron-withdrawing dicyanomethylene group in position 2.

Generally, the presence of electron-withdrawing groups at position 4 promotes higher bathochromic shifts than the electron-donating groups [37], but this effect is not so pronounced in the 4-styrylcoumarin derivatives (4 to 7), possibly due to the strong electron-withdrawing effect of the dicyanomethylene group in position 2. On the other hand, the molar extinction coefficients were strongly affected by the nature of EDGs or EWGs in para position at the 4-styryl group. The analysis of table 1 allows to verify that in the case of the 4-styrylcoumarin derivatives (4 to 7), EDGs promote high coefficients [e.g., ε (6) = 34000 cm⁻¹M⁻¹ vs ε (4) = 19000 cm⁻¹M⁻¹] and also high fluorescence quantum yields [e.g., ΦF (6) = 0.95 vs ΦF (4) = 0.04].

All coumarin derivatives exhibit large Stokes shifts due the extension of π-conjugated system in the molecule which is essential to an effective intramolecular charge transfer process of emissive excited state. The coumarin derivative (6) presents the higher fluorescence quantum yield, possibly due the presence of a strong electron donating amino substituent, but the decrease observed in the derivative (7) might be attributed to a substantial reduction of the oxygen atom electronic density in the presence of the dicyanomethylene electron-withdrawing substituent [38]. Fluorescent labels with large Stokes shifts values offer an advantage due the possible elimination of spectral overlap between absorption and emission, which reduces interference and also eliminates the quenching process, providing a very simple detection of the fluorescence emission. Fluorescent labels with large Stokes shifts are very important for Förster-type resonance energy-transfer (FRET) applications [39] and optical microscopy based on stimulated emission depletion (STED) [40].

### Theoretical calculations

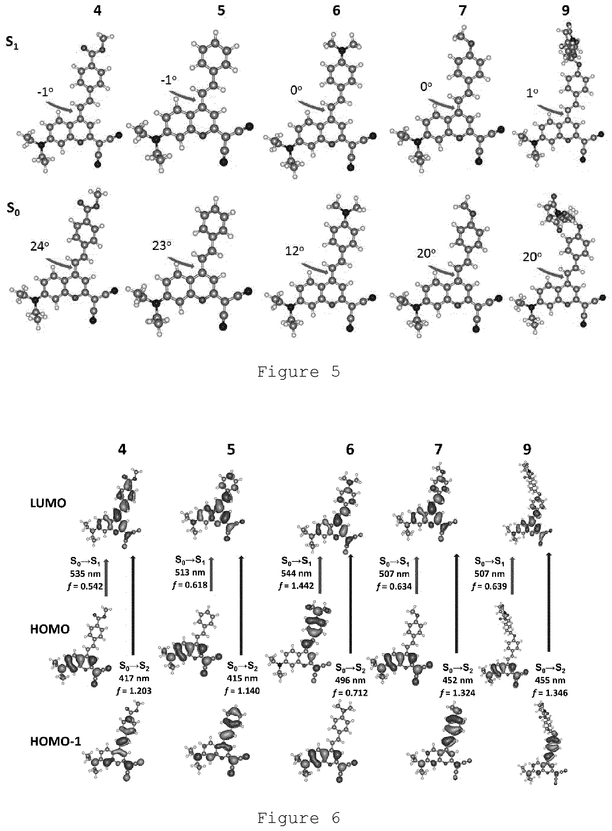

The optimized molecular geometry of the most relevant coumarin derivatives in both the ground and the first excited singlet state computed at PBE0/6-31G(d) level in acetonitrile (see computational section for more details), are depicted in Figure 5 and detailed in Table 2. In the electronic ground state, all the compounds present a deviation from planarity between the mean plane of the coumarin rings moiety and the 4-styryl group plane, restraining the π conjugation that links the donor and acceptor groups in the molecules. The ethylene bridge stays nearly in the plane of the benzyl group and the torsion occurs on the bond with the coumarin moiety. This dihedral angle is smaller for compound (6) (19.8°), 19.8° and 19.7° for (7) and (9) respectively and presents the higher values for compounds (4) (24.5°) and (5) (23.0°). For the excited state S1 however, a completely different picture emerges, with all the molecules becoming nearly planar with a minimum of -0.9° for compound (4) and maximum of 0.7° for compound (9). Another important geometrical property that is pertinent to access the electronic delocalization throughout the π-conjugation framework is the bond length alternation (BLA), the average length difference between a single and adjacent double bond. While the BLA of the compounds vary between 0.08 Å (compound 6) and 0.11 Å (compounds 4 and 5) in the ground S0 state, it considerably reduces to 0.04 Å for all compound in the S1 excited state. These significant differences in geometry between the relaxed S0 and S1 states, contributing for an enhancement of electronic delocalization and a decrease of the HOMO-LUMO energy gap, might justify a large Stokes shift where after a vertical excitation a significant structural relaxation of the excited state follows prior to emission [41,42]. The absorption wavelengths, oscillator strength (f) and the main components of lowest energy transitions of the compounds were calculated by Time Dependent Density Functional Theory (TDDFT) methods (Figure 6, Table 2). The lowest-energy excitations S1 and S2, despite presenting different oscillator strengths, share the same composition for all the compounds and are mainly of HOMO→LUMO and HOMO-1→LUMO character respectively (more than 98% in all cases).

**Table 2. Selected bond distances (Å) and dihedral angle (°) of the compounds in the ground S0 and excited S1 states.**

| | r[C10-C18] | r[C18 -C37] | r[C37 -C39] | BLA | Dₕ[C3-C10-C18-C37] |
|---|---|---|---|---|---|
| S₀ | | | | | |
| 4 | 1.456 | 1.348 | 1.459 | 0.11 | 24.5 |
| 5 | 1.454 | 1.349 | 1.458 | 0.11 | 23.0 |
| 6 | 1.442 | 1.359 | 1.440 | 0.08 | 11.9 |
| 7 | 1.450 | 1.353 | 1.451 | 0.10 | 19.8 |
| 9 | 1.450 | 1.353 | 1.451 | 0.10 | 19.6 |

| S₁ | | | | | |
|---|---|---|---|---|---|
| 4 | 1.409 | 1.382 | 1.432 | 0.04 | -0.9 |
| 5 | 1.409 | 1.380 | 1.440 | 0.04 | -0.6 |
| 6 | 1.417 | 1.385 | 1.430 | 0.04 | -0.3 |
| 7 | 1.406 | 1.383 | 1.436 | 0.04 | -0.5 |
| 9 | 1.405 | 1.383 | 1.436 | 0.04 | 0.7 |

The shape and spatial location of the states however is very different, which imply different characteristics of the intramolecular charge transfer that occurs upon the excitation. In most cases the HOMO and HOMO-1 are more localized either in the coumarin or the 4-styryl group, while the LUMO are less localized, extending over the bridging zone which facilitates low energy internal charge transfer absorptions [43]. The lowest energy excitation for compound (6), which exhibit the transitions with the higher oscillator strength, correspond to charge transfer from the 4-styryl group to the coumarin moiety, reflecting the strong donor capability of the amine group attach the 4-styryl group. For the other compounds, the S0→S1 excitation corresponds to the opposite charge transfers, with the HOMO state mainly located on the coumarin moiety and the LUMO spreading to the 4-styryl group. For these derivatives, however, this is not the most intense excitation, with the S0→S2 transition presenting much higher f values and thus dominating the absorption spectra. The donor character of compounds (7) and (9) attached units is evidenced by this transition that takes place between the HOMO-1 state, mostly located on the 4-styryl group, and the LUMO, mostly located on the coumarin moiety nitrile groups. Comparing the MOs of (7) and (9), they are comparable since the attached reactive group does not take part on the π-conjugation framework, thus resulting in very close spectroscopic properties. For most of the compounds, the calculated lowest energy transitions present good agreement with the experimental absorption maxima of the coumarin derivatives absorption (Table 1 and Table 3) with exception for compound (6). For this compound the experimental maximum at 496 nm can be assigned to the S0 → S2 transition, while the lowest energy S0 → S1 transition correspond to a visible shoulder located around 536 nm.

**Table 3. Calculated absorption data for the compounds and the main orbitals involved in the transitions**

| Compound 4 | | | |
|---|---|---|---|
| State | λ (nm) | f | Major MO → MO transitions |
| S1 | 534.7 | 0.542 | HOMO->LUMO (99%) |
| S2 | 417.4 | 1.203 | H-1->LUMO (98%) |
| S3 | 355.4 | 0.302 | H-2->LUMO (95%) |
| S4 | 344.8 | 0.314 | HOMO->L+1 (97%) |
| S5 | 303.9 | 0.037 | H-3->LUMO (93%) |
| S6 | 300.1 | 0.065 | H-4->LUMO (71%), HOMO->L+2 (21%) |
| S7 | 289.6 | 0.231 | H-1->L+1 (41%), HOMO->L+2 (42%) |
| S8 | 280.3 | 0.028 | H-4->LUMO (17%), H-1->L+1 (53%), HOMO->L+2 (22%) |
| S9 | 270.7 | 0.001 | H-1->L+5 (17%), HOMO->L+5 (77%) |
| S10 | 270.6 | 0.000 | H-5->LUMO (78%), H-5->L+1 (17%) |
| | | | |

| Compound 5 | | | |
|---|---|---|---|
| State | λ (nm) | f | Major MO → MO transitions |
| S1 | 513.3 | 0.618 | HOMO->LUMO (99%) |
| S2 | 415.0 | 1.140 | H-1->LUMO (98%) |
| S3 | 349.3 | 0.164 | H-2->LUMO (96%) |
| S4 | 316.2 | 0.244 | HOMO->L+1 (94%) |
| S5 | 304.1 | 0.023 | H-3->LUMO (95%) |
| S6 | 292.8 | 0.062 | H-4->LUMO (76%), HOMO->L+2 (17%) |
| S7 | 278.8 | 0.347 | H-4->LUMO (14%), H-1->L+1 (15%), HOMO->L+2 (61%) |
| S8 | 271.8 | 0.002 | H-1->L+5 (15%), HOMO->L+5 (76%) |
| S9 | 267.3 | 0.045 | H-1->L+1 (65%), HOMO->L+2 (15%) |
| S10 | 262.2 | 0.083 | H-1->L+1 (16%), HOMO->L+3 (10%), HOMO->L+4 (64%) |
| | | | |

| Compound 6 | | | |
|---|---|---|---|
| State | λ (nm) | f | Major MO → MO transitions |
| S1 | 543.9 | 1.442 | HOMO->LUMO (98%) |
| S2 | 495.6 | 0.712 | H-1->LUMO (99%) |
| S3 | 360.1 | 0.017 | H-2->LUMO (95%) |
| S4 | 317.1 | 0.130 | H-3->LUMO (24%), HOMO->L+1 (65%) |
| S5 | 310.8 | 0.054 | H-3->LUMO (39%), H-1->L+1 (25%), HOMO->L+1 (23%) |
| S6 | 306.1 | 0.242 | H-3->LUMO (31%), H-1->L+1 (63%) |
| S7 | 299.1 | 0.022 | H-4->LUMO (65%), HOMO->L+3 (20%) |
| S8 | 284.6 | 0.106 | H-4->LUMO (26%), HOMO->L+2 (40%), HOMO->L+3 (29%) |
| S9 | 280.6 | 0.272 | H-1->L+2 (13%), HOMO->L+2 (42%), HOMO->L+3 (31%) |
| S10 | 278.7 | 0.003 | H-2->L+5 (13%), H-1->L+5 (42%), HOMO->L+5 (38%) |

| Compound 7 | | | |
|---|---|---|---|
| State | λ (nm) | f | Major MO → MO transitions |
| S1 | 507.2 | 0.634 | HOMO->LUMO (99%) |
| S2 | 452.3 | 1.324 | H-1->LUMO (99%) |
| S3 | 354.3 | 0.034 | H-2->LUMO (96%) |
| S4 | 312.7 | 0.209 | HOMO->L+1 (88%) |
| S5 | 297.9 | 0.049 | H-3->LUMO (83%) |
| S6 | 293.6 | 0.031 | H-4->LUMO (78%) |
| S7 | 283.6 | 0.212 | H-4->LUMO (10%), H-1->L+1 (70%), HOMO->L+2 (15%) |
| S8 | 275.0 | 0.179 | H-1->L+1 (17%), HOMO->L+2 (66%) |
| S9 | 273.6 | 0.005 | H-2->L+5 (11%), HOMO->L+5 (75%) |
| S10 | 263.5 | 0.078 | H-1->L+2 (13%), HOMO->L+3 (31%), HOMO->L+4 (43%) |
| | | | |

| Compound 9 | | | |
|---|---|---|---|
| State | λ (nm) | f | Major MO → MO transitions |
| S1 | 506.8 | 0.639 | HOMO->LUMO (99%) |
| S2 | 455.2 | 1.346 | H-1->LUMO (99%) |
| S3 | 354.5 | 0.031 | H-2->LUMO (96%) |
| S4 | 312.5 | 0.206 | HOMO->L+1 (88%) |
| S5 | 298.1 | 0.052 | H-3->LUMO (80%) |
| S6 | 294.4 | 0.026 | H-4->LUMO (77%) |
| S7 | 284.5 | 0.208 | H-1->L+1 (73%), HOMO->L+3 (12%) |
| S8 | 275.1 | 0.198 | H-1->L+1 (14%), HOMO->L+3 (68%) |
| S9 | 273.8 | 0.005 | H-2->L+7 (11%), HOMO->L+7 (76%) |
| S10 | 266.7 | 0.000 | HOMO->L+2 (100%) |

### Methodology

### General Methods

All starting materials and reagents were analytical grade, purchased from Aldrich and used without further purification. The organic solvents were dried over appropriate drying agents and distilled prior to use. UV-Vis absorption spectra were recorded on the Thermo Electron Spectrophotometer Corporation, model Nicolet Evolution 300, using acetonitrile (CH₃CN) as solvent. Fluorescence spectra were recorded on a PerkinElmer Model LS 55 spectrophotometer. FTMS-ESI mass spectra were obtained on an Thermo Scientific Q Exactive Orbitrap Mass Spectrometer. Nuclear magnetic resonance (NMR) spectra were recorded at 400 MHz for ¹H NMR and at 100 MHz for ¹³C NMR, on a Brucker Advance III spectrometer. For NMR spectra, deuterated chloroform (CDCl₃) was used as solvent. The chemical shift (π) in ppm; coupling constants (J); relative intensity is indicated by the number of protons (H); multiplicities are indicated by singlet (s), doublet (d), double-doublet (dd), triplet (t), quadruple (q) and multiplet (m); coupling constants are given in Hertz (Hz).

### Procedures for the Preparation of Compounds 3 to 9

### 2-(7-(diethylamino)-4-methyl-2H-chromen-2-ylidene)malononitrile (3)

The synthesis of dicyanomethylenecoumarinmethyl derivative (3) was performed according to the described by Gandioso et al. [35].

### (E)-methyl 4-(2-(2-(dicyanomethylene)-7-(diethylamino)-2H-chromen-4-yl)benzoate (4)

A mixture of compound (3) (45 mg, 0.161 mmol, 1.0 eq), methyl 4-formylbenzoate (26 mg, 0.161 mmol, 1.0 eq) and piperidine (16 µL, 0.161 mmol, 1.0 eq) in dry acetonitrile (4 mL) was stirred at 40 °C, for a period of 2 h. The reaction was continuously monitored by TLC, using CH₂Cl₂ as eluent. After cooling to room temperature, the reaction mixture was evaporated to dryness and the residue was purified by flash chromatography, using CH₂Cl₂ as eluent, to yield methyl (E)-4-(2-(2-(dicyanomethylene)-7-(diethylamino)-2H-chromen-4yl)benzoate, as a dark red solid (48 mg, 71%). mp 252-254 °C. ¹H NMR (400 MHz, CDCl₃): 1.25 (6H, t, J = 7.1, N(CH₂CH₃)₂), 3.47 (4H, q, J = 7.1, N(CH₂CH₃)₂), 3.94 (3H, s, H-25), 6.59 (1H, d, J8,₆ = 2.4, H-8), 6.71 (1H, dd, J6,₅ = 9.2, J6,₈ = 2.4, H-6), 6.83 (1H, s, H-3), 7.40 (2H, sl, H-9, H17), 7.63 (1H, d, J5,₆ = 9.2, H-5), 7.64 (2H, d, J = 8.2, H-19, H-23), 8.08 (2H, d, J = 8.2, H-20, H22). ¹³C NMR (100 MHz, CDCl₃): 12.6 (C-11, C-13), 45.1 (C-10, C-12), 52.4 (C-25), 97.7 (C-8), 103.7 (C-3), 108.5 (C-4a), 110.7 (C-6), 114.6 (C-15, C-16), 115.6 (C-14), 122.2 (C-9), 125.8 (C-5), 127.7 (C-19, C-23), 130.4, (C-20, C-22), 131.1 (C-21), 137.4 (C-17), 139.6 (C-18), 146.0 (C-4), 151.8 (C-7), 155.5 (C-8a), 166.6 (C-24) 171.6 (C-2). FTMS(+) calc. for C₂₆H₂₄N₃O₃ [M+H]⁺ 426.1812 found 426.1807. UV λ^{max} (nm, CH₃CN): 312, 348, 395, 520. ε (cm⁻¹ M⁻¹): 19000. Φ_{F} = 0.04.

### (E)-2-(7-(diethylamino)-4-styryl)-2H-chromen-2-ylidene) malononitrile (5)

A mixture of compound (3) (100 mg, 0.358 mmol, 1.0 eq), benzaldehyde (36 µL, 0.358 mmol, 1.0 eq) and piperidine (36 µL, 0.358 mmol, 1.0 eq) in dry acetonitrile (4 mL), was stirred at 85 °C, for a period of 5 minutes. The reaction was continuously monitored by TLC, using CH₂Cl₂/hexane (8:2) as eluent. After cooling at room temperature, the reaction mixture was evaporated to dryness and the residue purified by flash chromatography, using CH₂Cl₂/hexane (8:2) as eluent, to yield (E)-2-(7-(diethylamino)-4-styryl)-2H-chromen-2ylidene) malononitrile, as a red solid (121 mg, 92%) . mp 162-164 °C ¹H NMR (400 MHz, CDCl₃): 1.25 (6H, t, J = 7.1, N(CH₂CH₃)₂), 3.47 (4H, q, J = 7.1, N(CH₂CH₃)₂), 6.61 (1H, d, J8,₆ = 2.4, H-8), 6.70 (1H, dd, J6,₅ = 9.1, J6,8 = 2.4, H-6), 6.87 (1H, s, H-3), 7.34 (1H, d, J9,₁₇ = 16, H-9), 7.40-7.46 (4H, m, H-17, H-20, H-22, H-21), 7.60 (2H, d, J19,₂₀ = 6.8, H-19, H-23), 7.65 (1H, d, J5,₆ = 9.1, H-5) . ¹³C NMR (100 MHz, CDCl₃): 12.5 (C-11, C-13), 45.0 (C-10, C-12), 97.6 (C-8), 103.1 (C-3), 108.6 (C-4a), 110.6 (C-6), 114.8 (C-15, C-16), 115.7 (C-14), 119.5 (C-9), 125.7 (C-5), 127.8 (C-19, C-23), 129.1 (C-20, C-22), 130.1 (C-21), 135.4 (C-18), 138.9 (C-17), 146.5 (C-4), 151.6 (C7), 155.4 (C-8a), 171.6 (C-2). FTMS(+) calc. for C₂₄H₂₂N₃O [M+H]⁺ 368.1757 found 368.1752. UV λ^{max} (nm, CH3CN): 341, 396, 516. ε (cm⁻¹ M⁻¹): 17000. Φ_{F} = 0.16.

### (E)-2-(7-(diethylamino)-4-(4-(dimethylamine)styryl)-2H-chromen-2ylidene)malononitrile (6)

A mixture of compound (3) (150 mg, 0.537 mmol, 1.0 eq), 4-(dimethylamine)benzaldehyde (81 mg, 0.537 mmol, 1.0 eq) and piperidine (60 µl, 0.536 mmol, 1.0 eq), in dry acetonitrile (4 mL), was stirred at 85°C, for a period of 24h. The reaction was continuously monitored by TLC, using CH₂Cl₂ as eluent. After cooling to room temperature, the reaction mixture was evaporated to dryness and the residue was purified by flash chromatography, using as eluents CH₂Cl₂/hexane (8:2), CH₂Cl₂/hexane (9:1), and CH₂Cl₂ to yield (E)-2-(7-(diethylamino)-4-(4-(dimethylamine)styryl)-2H-chromen-2ylidene)malononitrile, as a red solid (210 mg, 95%) . mp 214-216 °C ¹H NMR (400 MHz, CDCl₃): 1.24 (6H, t, J = 7.1, N(CH₂CH₃)₂), 3.06 (6H, s, 2xNCH₃), 3.45 (4H, q, J = 7.1, N(CH₂CH₃)₂), 6.54 (1H, d, J8,₆ = 2.4, H-8), 6.68 (2H, d, J20,₁₉ = 8.8, H-20, H-22), 6.69 (1H, dd, J6,₅ = 8.8, J6,₈ = 2.4, H-6), 6.77 (1H, s, H-3), 7.08 (1H, d, J 9,₁₇ = 15.8, H-9), 7.37 (1H, d, J 17,₉ = 15.8, H17), 7.47 (1H, d, J19,₂₀ = 8.8, H-19, H-23), 7.66 (1H, d, J5,₆ = 8.8, H-5). ¹³C NMR (100 MHz, CDCl₃): 12.7 (C-11, C-13), 40.3 (C-24, C-25), 45.0 (C-10, C-12), 97.6 (C-8), 100.8 (C-3), 108.8 (C-4a), 110.5 (C-6), 112.1 (C-20, C-22), 113.2 (C-9), 115.7 (C-14), 116.6 (C-15, C-16), 123.3 (C-18) 125.6 (C-5), 129.9 (C-19, C-23), 140.0 (C-17), 147.3 (C-4), 151.5 (C-7), 151.8 (C-21), 155.5 (C-8a), 171.3 (C-2). FTMS(+) calc. for C₂₆H₂₇N₄O [M+H]⁺ 411.2179 found 411.2174. UV λ^{max} (nm, CH₃CN): 294, 341, 496. ε (cm⁻¹ M⁻¹): 34000. Φ_{F} = 0.95.

### (E)-2-(7-(diethylamino)-4-(4-methoxystyryl)-2H-chromen-2-ylidene)malononitrile (7)

A mixture of compound (3) (279 mg, 0.251 mmol, 1.0 eq), 4-methoxybenzaldehyde (30 µL, 0.251 mmol, 1.0 eq) and piperidine (25 µl, 0.251 mmol, 1.0 eq) in dry acetonitrile (5 mL), was stirred at 85°C, for a period of 17h. The reaction was continuously monitored by TLC, using CHCl₃/CH₃OH (99:1) as eluent. After cooling to room temperature, the reaction mixture was evaporated to dryness and the residue was purified by flash chromatography, using as eluent CHCl₃/CH₃OH (99:1) to yield (E)-2-(7-(diethylamino)-4-(4-metoxystyryl)-2Hchromen-2-ylidene) malononitrile, as a red solid (95 mg, 96%). mp 230-232 °C ¹H NMR (400 MHz, CDCl₃): 1.24 (6H, t, J = 7.1, N(CH₂CH₃)₂), 3.46 (4H, q, J = 7.1, N(CH₂CH₃)₂), 3.87 (3H, s, H-24), 6.58 (1H, d, J8,₆ = 2.4, H-8), 6.70 (1H, dd, J6,₅ = 9.1, J6,₈ = 2.4, H-6), 6.82 (1H, s, H-3), 6.94 (2H, d, J20,₁₉ = 8.7, H-20, H-22), 7.19 (1H, d, J 9,₁₇ = 15.9, H-9), 7.39 (1H, d, J 17,₉ = 15.9, H-17), 7.54 (1H, d, J19,₂₀ = 8.7, H-19, H-23), 7.65 (1H, d, J5,₆ = 9.1, H-5) . ¹³C NMR (100 MHz, CDCl₃): 12.6 (C-11, C-13), 45.1 (C-10, C-12), 55.6 (C-24), 97.7 (C-8), 102.4 (C-3), 108.7 (C-4a), 110.6 (C-6), 114.7 (C-20, C-22), 115.1 (C-14), 116.1 (C-15, C-16), 116.9 (C-9), 125.8 (C-5), 128.3 (C-18), 129.6 (C-19, C-23), 138.8 (C-17), 146.9 (C-4), 151.7 (C-7), 155.6 (C-8a), 161.5 (C-21), 171.6 (C-2). FTMS (+) calc. for C₂₅H₂₄O₂N₃ [M+H]⁺ 398.1863 found 398.1854. UV λ^{max} (nm, CH₃CN): 418, 522. ε (cm⁻¹ M⁻¹): 24000. Φ_{F} = 0.20.

### (E)-6-(4-(2-(2-(dicyanomethylene)-7-(diethylamino)-2H-chromen-4yl)vinyl)phenoxy)hexanoic acid (8)

A mixture of compound (3) (125 mg, 0.448 mmol, 1.0 eq), 6-(4-formylphenoxy)hexanoic acid (106 mg, 0.448 mmol, 1.0 eq) and piperidine (44 pL, 0.448 mmol, 1.0 eq) in dry acetonitrile (10 mL), was stirred at 85°C for 24 h. The reaction was monitored by TLC using CHCl₃/CH₃OH (95:5) as eluent. After cooling to room temperature, the reaction mixture was evaporated to dryness and the residue was purified by flash chromatography, using CHCl₃/CH₃OH (99:1) and CHCl₃/CH₃OH (95:5) as eluents, to yield (E)-6-(4-(2-(2(dicyanomethylene)-7-(diethylamino)-2H-chromen-4-yl)vinyl)phenoxy)hexanoic acid , as a red solid (187 mg, 84%). ¹H NMR (400 MHz, CDCl₃): 1.25 (6H, t, J = 6.8, N(CH₂CH₃)₂), 1.56 (2H, m, H-26), 1.74 (2H, m, H-27), 1.84 (2H, m, H-25), 2.42 (2H, t, J = 6.6, H-28) 3.46 (4H, q, J = 6.8, N(CH₂CH₃)₂), 4.02 (2H, t, H-24), 6.58 (1H, sl, H-8), 6.70 (1H, d, J6,₅ = 9.2, H-6), 6.83 (1H, s, H-3), 6.93 (2H, d, J20,₁₉ = 7.8, H-20, H-22), 7.19 (1H, d, J9,₁₇ = 15.8, H-9), 7.39 (1H, d, J17,₉ = 15.8, H-17), 7.53 (2H, d, J19,₂₀ = 7.8, H-19, H-23), 7.65 (1H, d, J5,₆ = 9.2, H-5).¹³C NMR (100 MHz, CDCl₃): 12.7 (C-11, C-13), 24.5 (C-27), 25.7 (C-26), 29.0 (C-25), 33.8 (C-28), 45.1 (C-10, C-12), 68.0 (C-24), 97.7 (C-8), 102.3 (C-3), 108.7 (C-4a), 110,6 (C-6), 115.1 (C-14), 115.2 (C-20, C-22), 116.1 (2xCN), 116.8 (C-9), 125.8 (C-5), 128.2 (C-18), 129.6 (C-19, C-23), 138.9 (C-17), 147.0 (C4), 151.7 (C-7), 155.6 (C-8a), 160.9 (C-21), 171.6 (C-29), 178.7 (C-2). FTMS(+) calc. for C₃₀H₃₂N₃O₄ [M+H]⁺ 498.2387 found 498.2379. UV λ^{max} (nm, CH3CN): 340, 418, 519. ε (cm⁻¹ M¹): 24000. Φ_{F} = 0.28.

### (E)-2,5-dioxopyrrolidin-1-yl 6-(4-(2-(2-(dicyanomethylene)-7-(diethylamino)-2Hchromen-4-yl)vinyl)phenoxy)hexanoate (9)

A mixture of compound (8) (110 mg, 0.221 mmol, 1.0 eq), DCC (55 mg, 0.265 mmol, 1.2 eq), DMAP (3 mg, 0.0221 mol, 10 %) in dry DMF (2 mL) and dry acetonitrile (25 mL), was stirred at room temperature for 5 minutes. After this period N-hydroxysuccinimide (28 mg, 0.243 mmol, 1.2 eq) was added and the reaction was stirred at room temperature for 5 h. The reaction was monitored by TLC using CHCl₃/CH₃OH (95:5) as eluent. The reaction mixture was evaporated to dryness and the residue was purified by flash chromatography using CH₃CN/CHCl₃ (7:3) as eluents, to yield (E)-2,5-dioxopyrrolidin-1-yl 6-(4-(2-(2(dicianomethylene)-7-(diethylamino)-2H-chromen-4-yl)vinyl)phenoxy)hexanoate, as a red solid (117 mg, 89%). mp 192-194 °C ¹H NMR (400 MHz, CDCl₃): 1.24 (6H, t, J = 6.8, N(CH2CH3)2), 1.58-1.66 (2H, m, H-26), 1.83-1.88 (4H, m, H-25, H-27), 2.67 (2H, t, J = 7.4, H28), 2.85 (2H, m, H-31, H-32), 3.46 (4H, q, J = 6.8, N(CH₂CH₃)₂), 4.02 (2H, t, J = 6.4, H-24), 6.59 (1H, d, J8,₆ = 2.4, H-8), 6.70 (1H, dd, J6,₅ = 9.2, J6,₈ = 2.4, H-6), 6.85 (1H, s, H-3), 6.93 (2H, d, J20,₁₉ = 8.6, H-20, H-22), 7.20 (1H, d, J9,₁₇ = 16.0, H-9), 7.40 (1H, d, J17,₉ = 16.0, H-17), 7.54 (2H, d, J19,₂₀ = 8.6, H-19, H-23), 7.66 (1H, d, J6,₅ = 9.2, H-5). ¹³C NMR (100 MHz, CDCl₃): 12.6 (C-11, C-13), 24.5 (C-27), 25.4 (C-26), 25.7 (C-31, C-32), 28.8 (C-25), 31.0 (C-28), 45.1 (C-10, C-12), 67.8 (C24), 97.7 (C-8), 102.4 (C-3), 108.8 (C-4a), 110,6 (C-6), 115.2 (C-14, C-20, C-22), 116.8 (2xCN, C9), 125.8 (C-5), 128.2 (C-18), 129.6 (C-19, C-23), 138.9 (C-17), 147.0 (C-4), 151.7 (C-7), 155.6 (C8a), 160.9 (C-21), 168.6 (C-29), 169.3 (C-30, C-33), 171.7 (C-2) . FTMS (+) calc. for C₃₄H₃₅N₄O₆ [M+H]⁺ 595.2551 found 595.2543. UV λ^{max} (nm, CH₃CN): 340, 418, 523. ε (cm⁻¹ M⁻¹): 24000. Φ_{F} = 0.29.

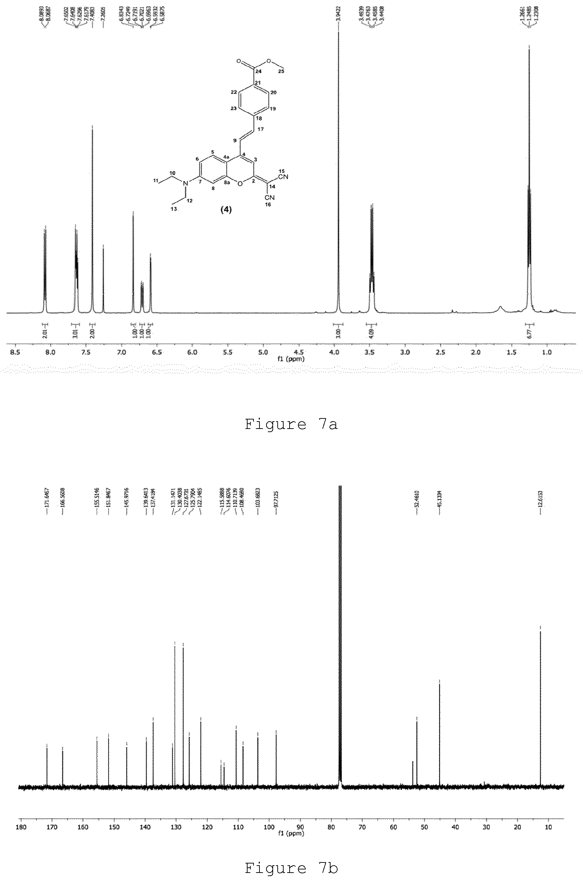

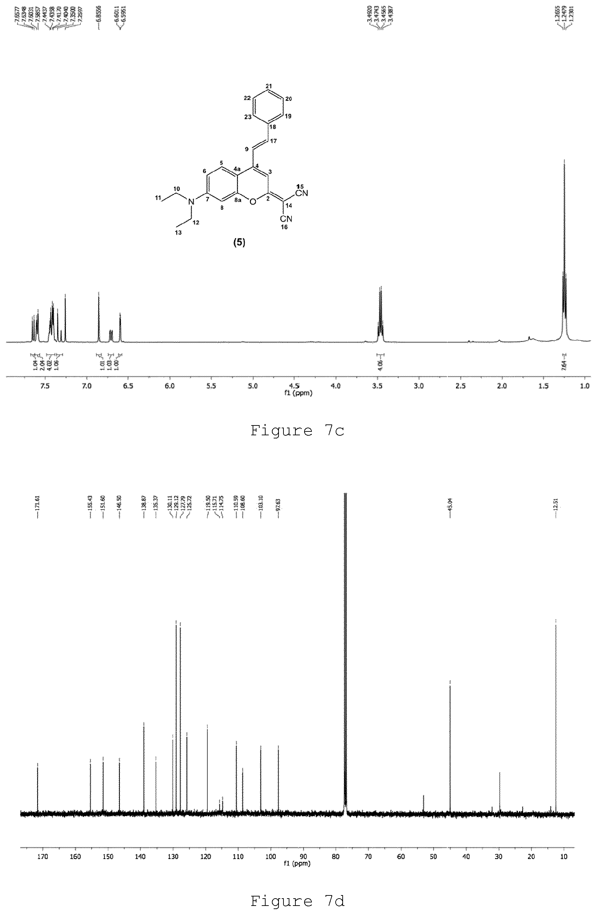

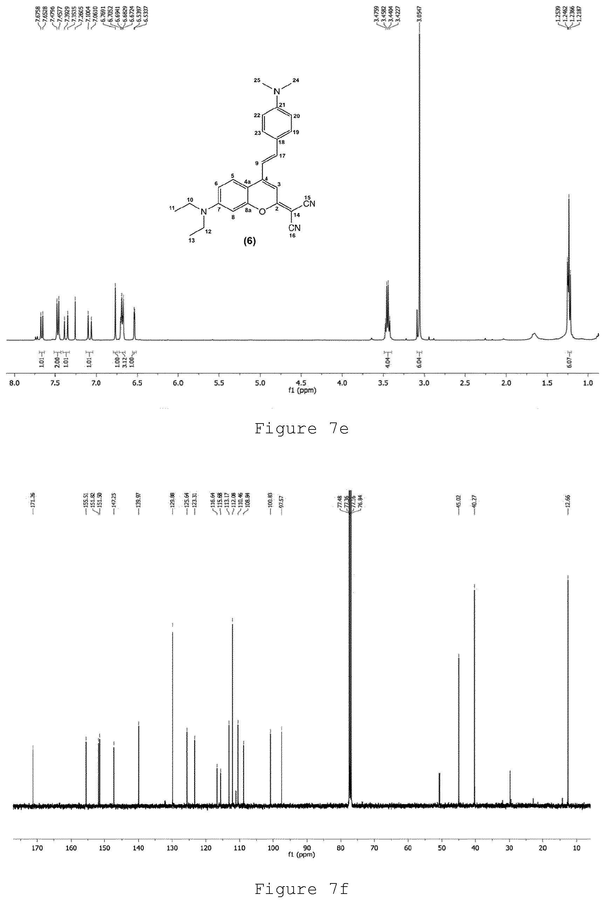

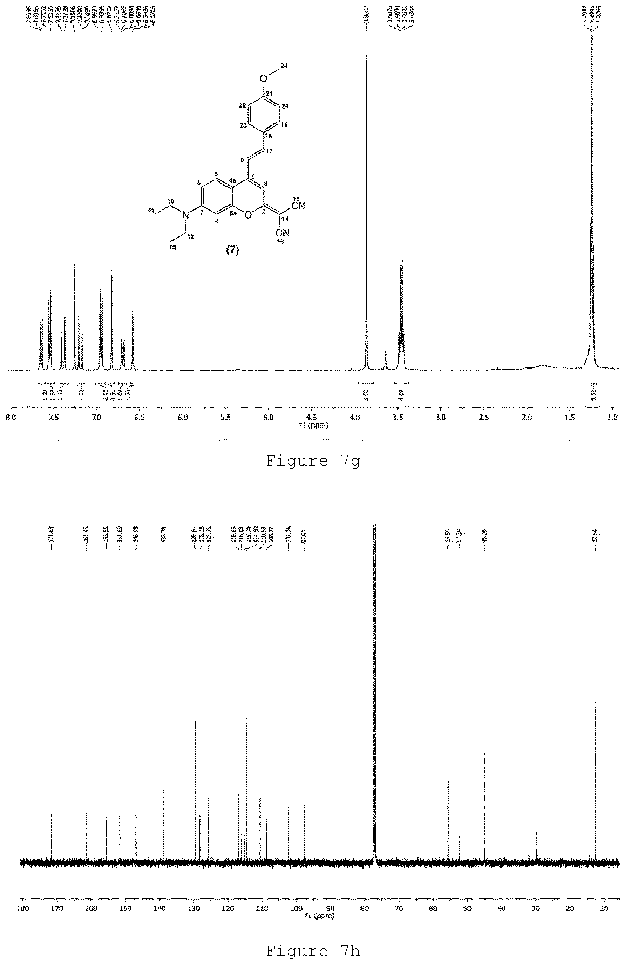

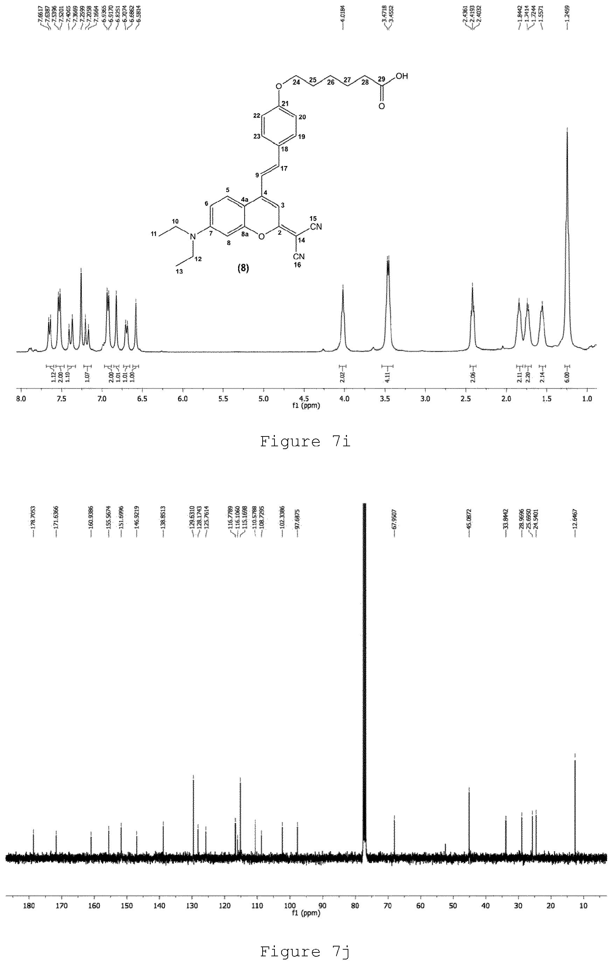

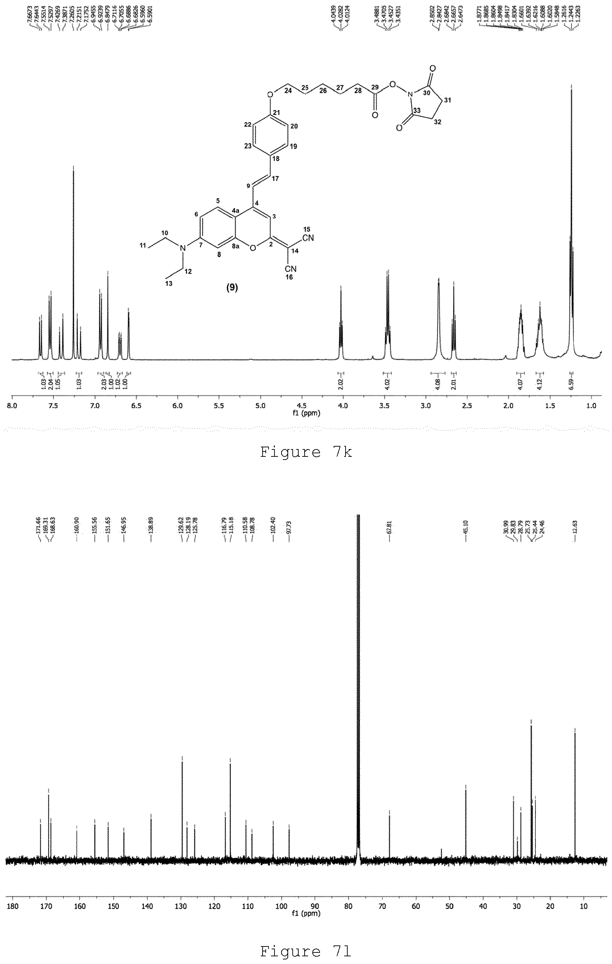

Figure 7a to 7l show the 1H and 13C NMR and HRMS spectra of the 4-styrylcoumarin derivatives (4 to 9).

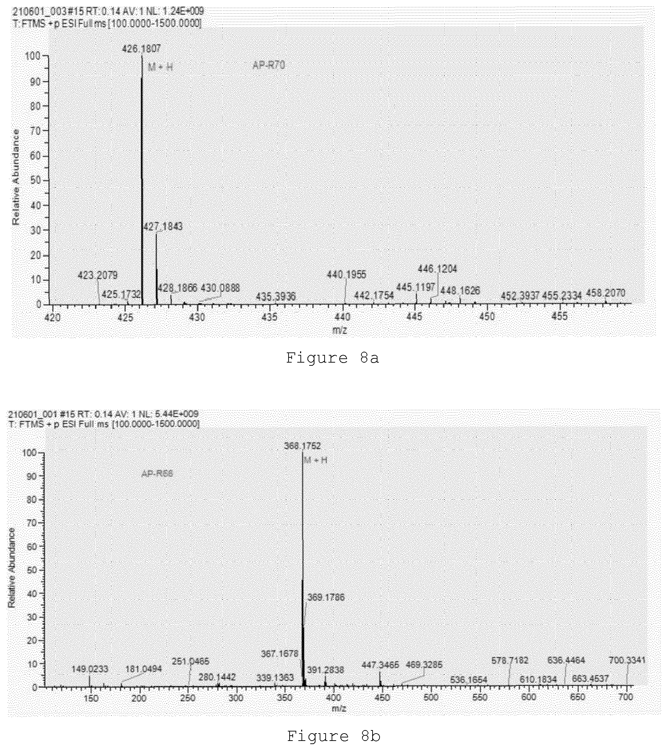

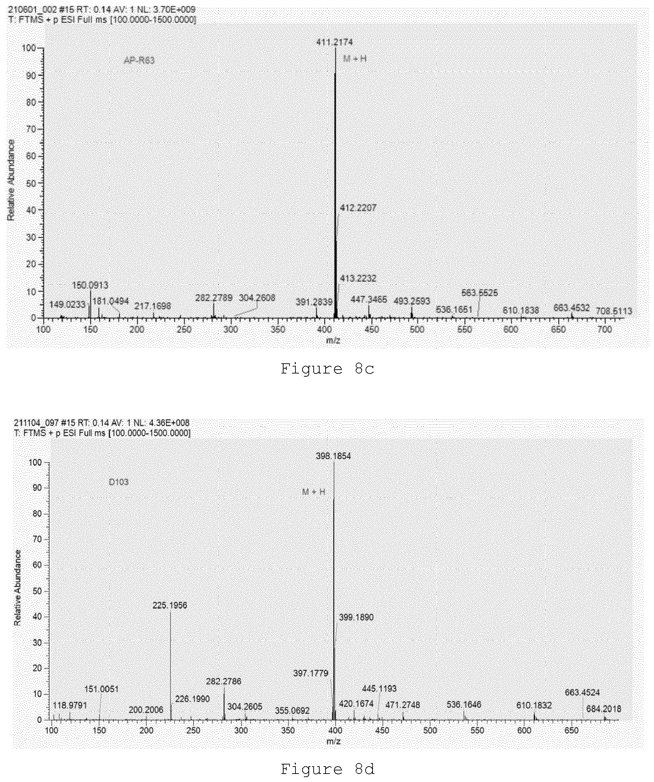

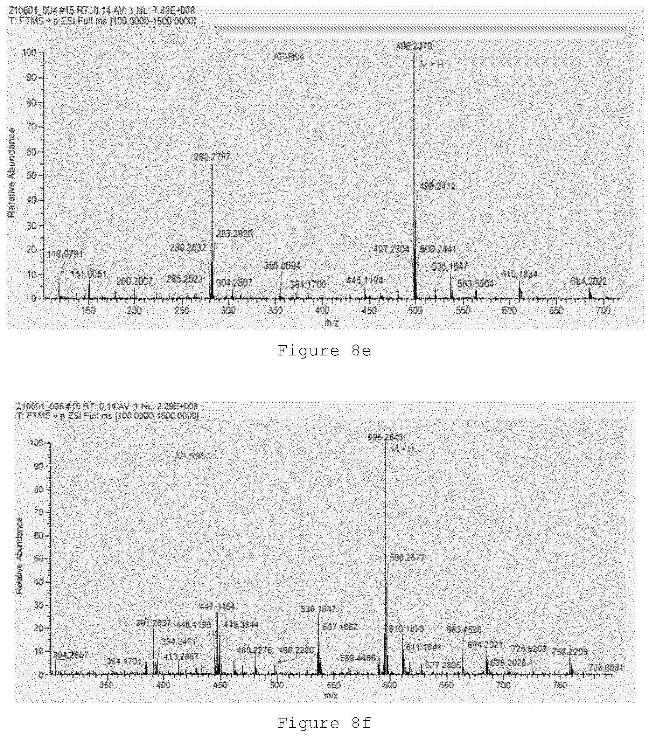

Figure 8a to 8f show the mass spectra of the 4-styrylcoumarin derivatives (4 to 9).

### Quantum chemical calculations

Quantum calculations were performed with the Gaussian 16 software package [44]. The hybrid PBE0 functional [45] with the standard 6-31G(d,p) basis set was used for geometry optimizations, both in the ground state and in the first singlet excited state, while the larger 6-311+G(d,p) basis set was employed for the spectra calculations. The PBE0 functional, besides being widely used for calculating ground state geometries, proved also to be very effective for TD-DFT calculation of excited-state structures [46]. In all cases solvent effects were taken into account by means of the implicit polarized continuum model (PCM) [47, 48] No symmetry constraints are used during geometry optimizations. The frequency analysis confirmed the optimized structures as minima presenting all real valued frequencies.

Table 4 shows the emission data for the compounds.

**Table 4. Calculated emission data for the studied compounds and the main orbitals involved in the S1→S0 transitions.**

| Compound | λ (nm) | f | Major MO → MO transitions |
|---|---|---|---|
| 4 | 630.2 | 0.438 | HOMO->LUMO (99%) |
| 5 | 590.0 | 0.505 | HOMO->LUMO (99%) |
| 6 | 583.0 | 1.512 | HOMO->LUMO (99%) |
| 7 | 577.1 | 0.539 | HOMO->LUMO (99%) |
| 9 | 576.2 | 0.546 | HOMO->LUMO (99%) |

### Example 2

### Results

A series of novel 2-piperidinium-4-styrylcoumarin derivatives (Formula II and IV), with large Stokes shifts and high fluorescence quantum yields, were synthesised using an efficient and low-cost synthetic strategy, as potential fluorescent labels for biomolecules. Density functional theory and time dependent density functional theory calculations were conducted in order to rationalize the observed photophysical properties.

### Synthesis

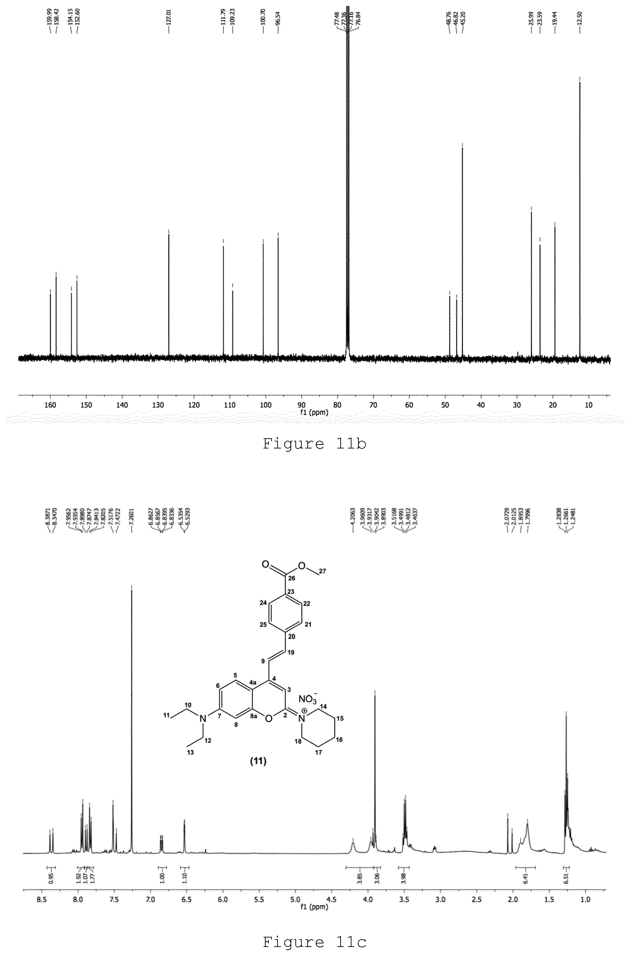

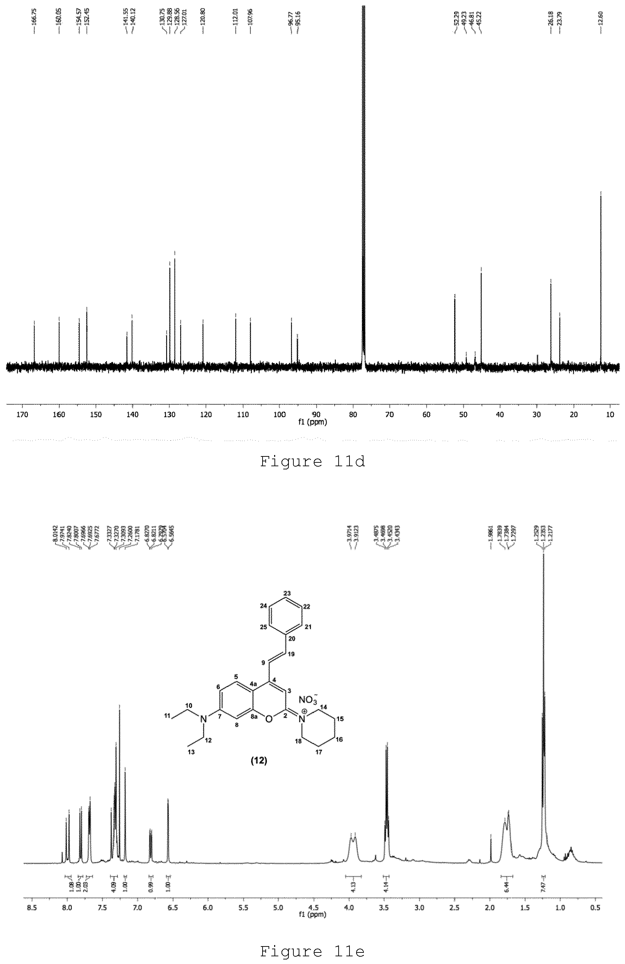

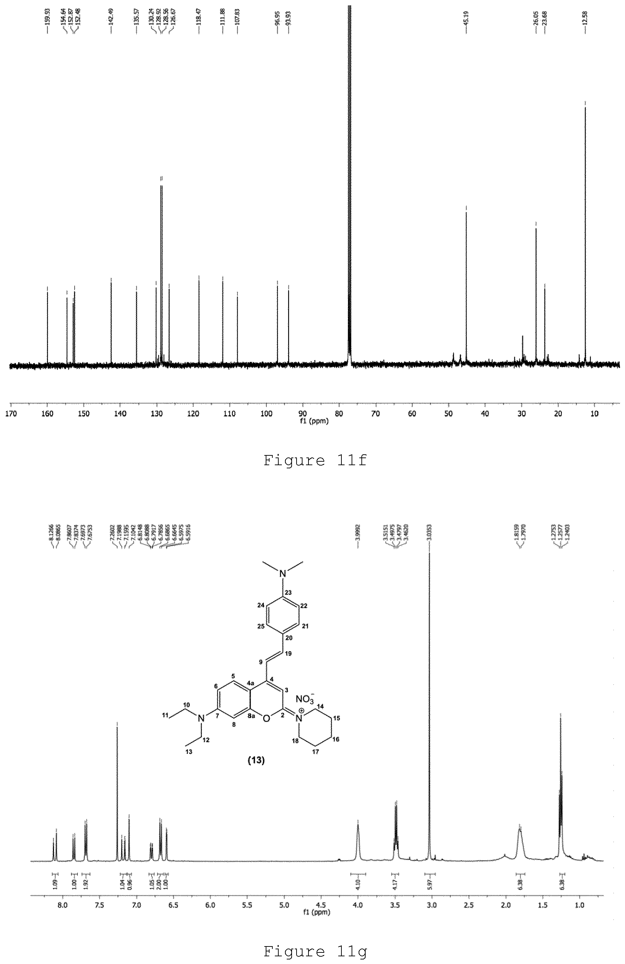

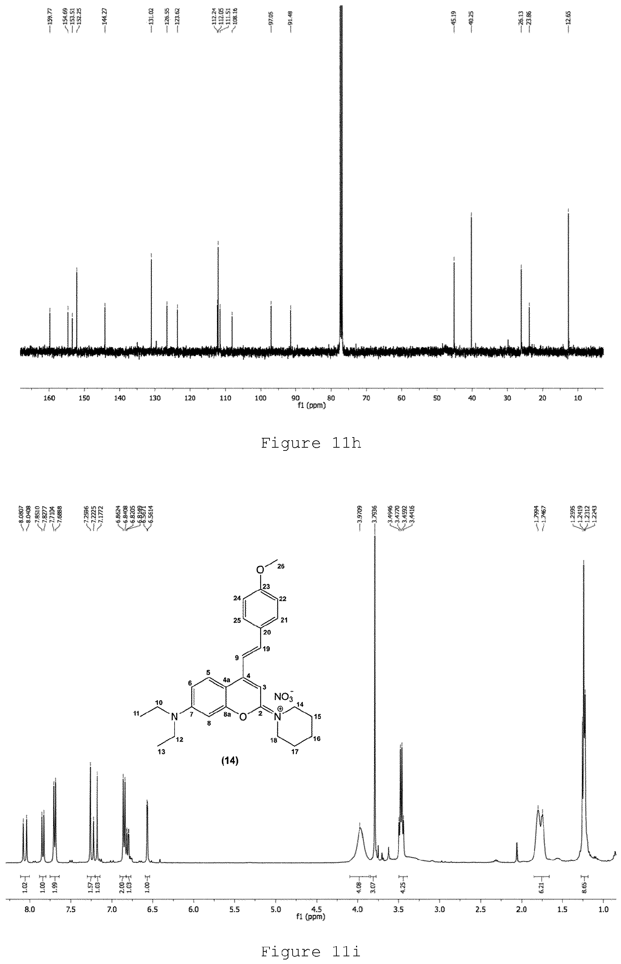

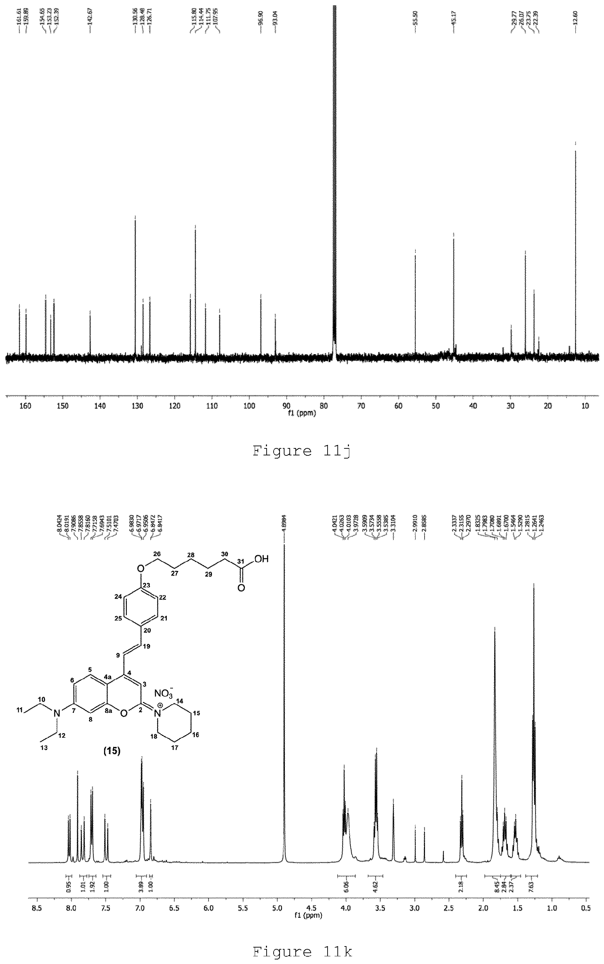

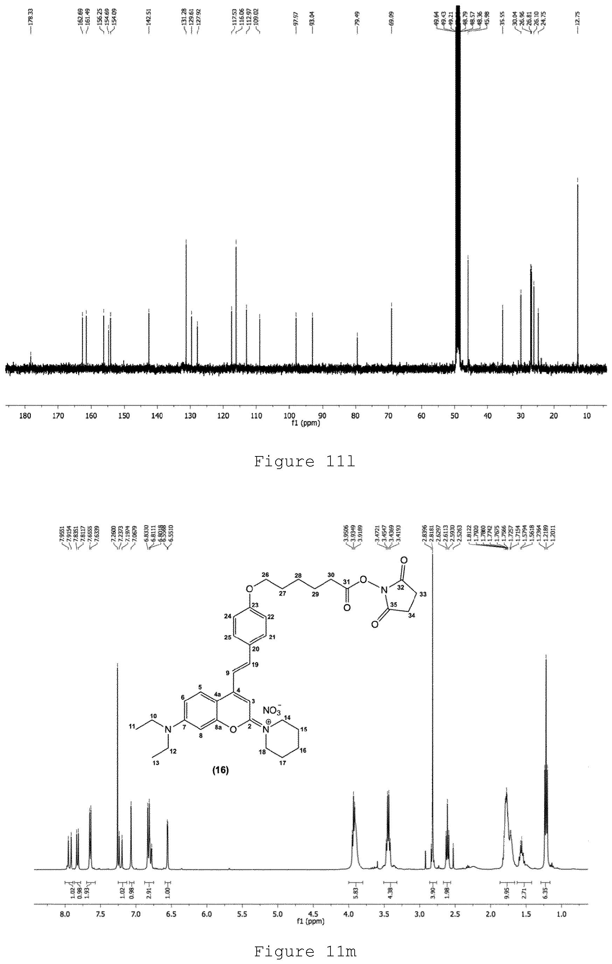

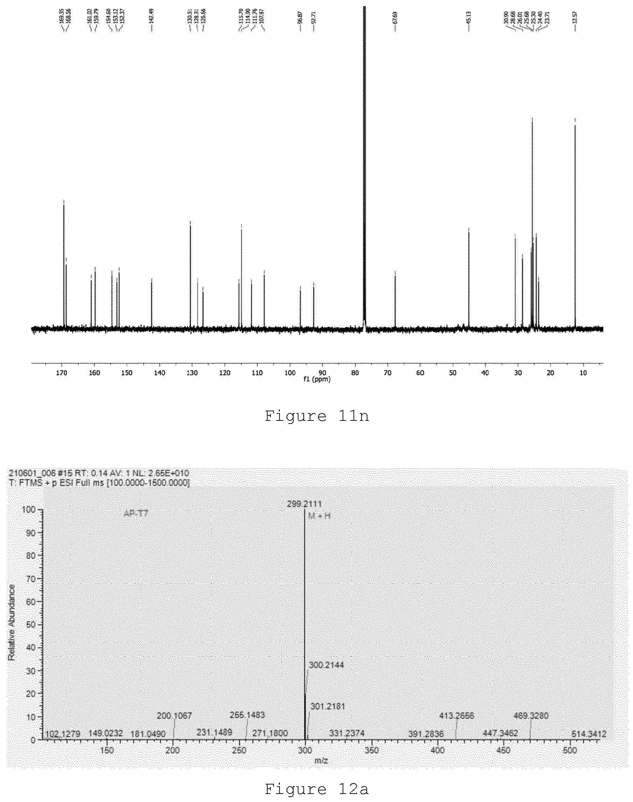

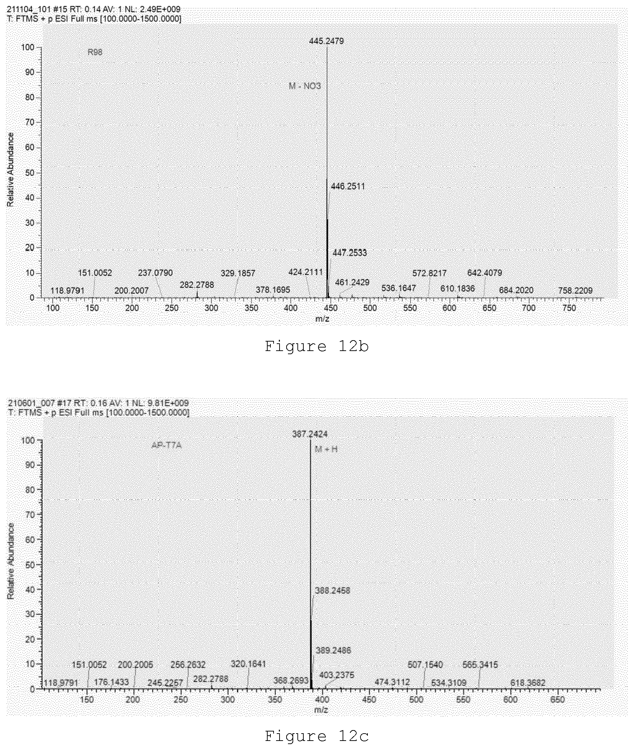

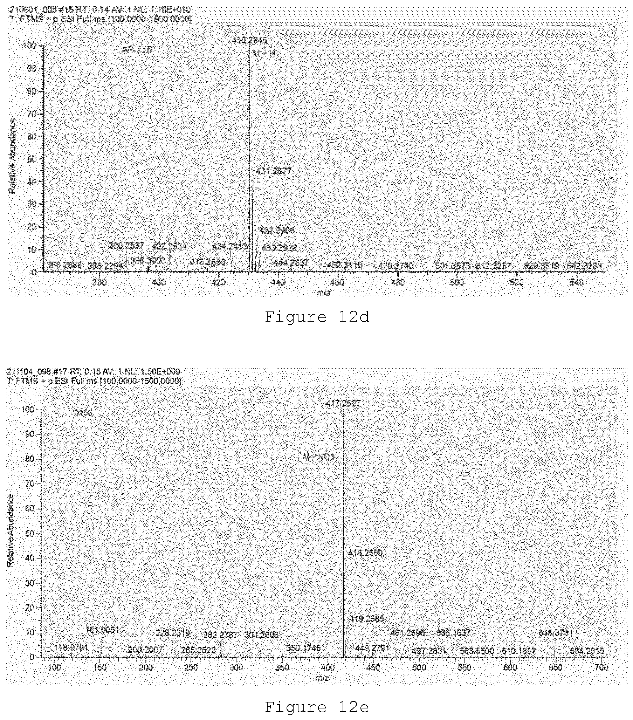

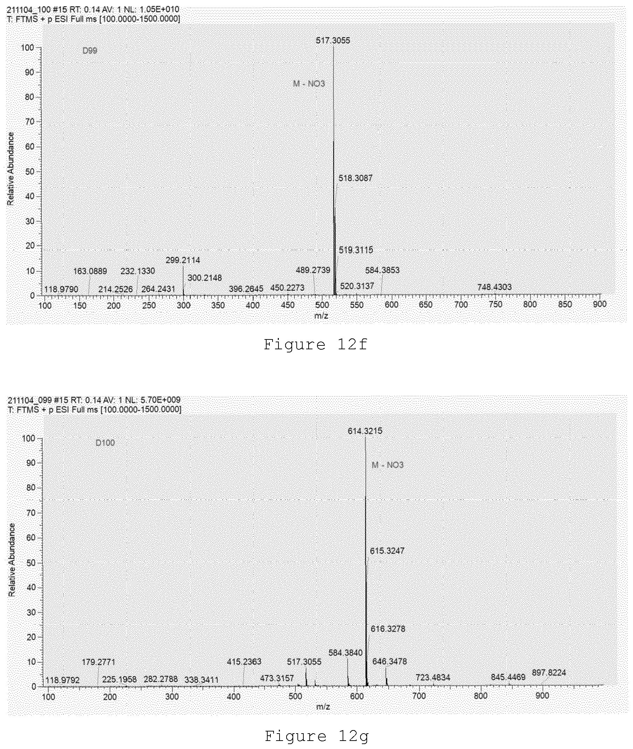

The key molecule to the synthesis of these new red-shifted chromophores is the 2-piperidinium coumarin derivative (10), that was easily obtained after the thionation reaction in the carbonyl group of the 7-diethylamino-4-methylcoumarin (1) [35] followed by condensation of such thionated coumarin (2) with piperidine (Figure 9), in very good yield (85%). This very interesting new "green" molecule (Figure 10), exhibit a higher bathochromic shift than 66 nm when compared with its precursor (1). Additionally, and with the aim of increasing the *π* delocalization and obviously the push-pull character of the molecule, we idealized the inclusion of one additional styryl group in position 4 in the referred 2-piperidinium coumarin derivative (10). The presence of electrondonating groups (EDGs) or electron-withdrawing groups (EWGs) at para position, in this 4-styryl group with can improve the photophysical properties of the respective derivatives, such as higher bathochromic and large Stokes shifts, as well as high fluorescence quantum yields. According to the aforementioned, we describe the synthesis and spectroscopic characterization of a small library of 2-piperidinium-4-styrylcoumarin derivatives to explore the effect of EDGs and EWGs on the photophysical properties of the new chromophores. Additionally, considering the binomial photophysical properties and cost, one of the best candidates has been functionalized as an effective fluorescent label for biomolecules through a reactive succinimidyl ester group. The main synthetic strategy to synthesize the 2-piperidinium coumarin derivatives was outlined considering the possible aldol condensation reactions at position 4 due to the high acidity of the methyl protons present in 1-(7-(diethylamino)-4-methyl-2H-chromen-2-ylidene)piperidin-1-ium nitrate (10). The synthetic routes followed for the preparation of the novel 2-piperidinium coumarin derivatives (11 to 14) are shown in Figure 9. The 2-piperidinium-4-styrylcoumarin derivatives (11 to 14) were obtained through highly efficient and stereoselective aldol condensation reactions between the aromatic aldehydes (methyl 4-formylbenzoate, benzaldehyde, 4-(dimethylamino)benzaldehyde and 4-methoxybenzaldehyde) and the 2-piperidinium coumarin derivative (10), in good to high yields. The abovementioned new products (Figure 10) are easily isolated after silica column chromatography and were fully characterized by NMR spectroscopy (Figure 11a to 11n) and HRMS (SI) (and Figures 12a to 12g)). All spectral data were consistent with the proposed structures.

### Photophysical properties

The photophysical properties of all synthesised coumarin derivatives was studied, and their absorption and emission properties, as well as fluorescence quantum yields, are summarised in Table 5. The absorption and emission spectra of the of the new 2-piperidinium-4-styrylcoumarin derivatives (10 to 14) are displayed in Figure 13. Almost all 2-piperidinium-4-styrylcoumarin derivatives (10 to 13), exhibit absorption and emission maxima at longer wavelengths, when compared to intermediate, 2-piperidinium coumarin derivative (10), possibly due to the intramolecular charge transfer effect between the coumarin electron-donating NEt2 group conjugated with the styryl groups in position 4 and the electron-withdrawing piperidinium group in position 2. The derivatives 14, 15 and 16, with an oxygen as an electron donor atom in the 4-styryl group have a different behaviour from other derivatives, with a hypsochromic shift in the absorption spectrum but with a bathochromic shift in the emission spectrum, what gives them large Stokes shifts (Table 5). In general, all the coumarin derivatives exhibit large Stokes shifts due the extension of π-conjugated system in the molecule which is essential to an effective intramolecular charge transfer process of emissive excited state. The possible elimination of the spectral overlap between absorption and emission, in fluorophores with large Stokes shifts, allows to eliminate the quenching process and reduce interference, providing an effective detection of the fluorescence emission. Generally, the presence of electron-withdrawing groups at position 4 promotes higher bathochromic shifts than the electron-donating groups [37], but this effect is not observed in the 2-piperidinium-4-styrylcoumarin derivatives (11 to 14), possibly due to the strong electron-withdrawing effect of the piperidinium positive charge in position 2. On the other hand, the molar extinction coefficients were strongly affected by the nature of EDGs or EWGs in para position at the 4-styryl group. The analysis of table 5 allows to verify that in the case of the 2-piperidinium-4-styrylcoumarin derivatives (11 to 14), EDGs promote high coefficients [e.g., ε (13) = 39000 cm⁻¹M⁻¹ vs ε (11) = 18000 cm⁻¹M⁻¹] and also high fluorescence quantum yields [e.g., ΦF (6) = 0.98 vs ΦF (4) = 0.08]. The extension of π-conjugated system generally promotes an effective intramolecular charge transfer process what can cause the decrease of fluorescence quantum yield in the molecules [49]. This effect is not observed for compounds 13 and 14, as they present high fluorescence quantum yields, possibly due the presence of strong electron donating substituents in para position of the styryl groups [40]. Considering the large Stokes shift and the high fluorescence quantum yield induced by the methoxy group in the 2-piperidinium-4-styrylcoumarin derivative (14) (Table 5), we selected this electron-donating substituent to synthesize an effective and inexpensive fluorescent label for biomolecules. The aldol condensation of (10) with the 6-(4-formylphenoxy)hexanoic acid afforded the derivative (15) which was further reacted with N-hydroxysuccinimide to obtain the fluorescent label (16) (Figure 14). The referred fluorescent label can be obtained through five linear and effective synthetic steps from inexpensive commercially available precursors and as expected, its photophysical properties are very similar to the derivative (14) .

**Table 5. Spectroscopic properties of 7-diethylamino-4-methyl-coumarin derivatives.**

| **Compound** | **λ_{abs}^{a} (nm)** | **λₑₘ^{b} (nm)** | **Stokes shift (nm, cm⁻¹)** | **ε^{c} (cm⁻¹m⁻¹)** | **Φ_{F}^{d}** |
|---|---|---|---|---|---|
| **1** | 371 | 434 | 63, 3913 | 22910 | 0.73 |
| **10** | 437 | 490 | 53, 2475 | 21000 | 0.14 |
| **11** | 484 | 609 | 125, 4241 | 18000 | 0.08 |
| **12** | 467 | 525 | 58, 2366 | 17000 | 0.34 |
| **13** | 485 | 608 | 123, 4171 | 39000 | 0.98 |
| **14** | 410 | 578 | 168, 7089 | 30000 | 0.82 |
| **15** | 406 | 578 | 172, 7330 | 30000 | 0.90 |
| **16** | 404 | 577 | 173, 7421 | 30000 | 0.92 |

| | | | | | |
|---|---|---|---|---|---|
| ^{a} Absorption maxima in acetonitrile. ^{b} Emission maxima in acetonitrile. ^{c} Molar extinction coefficient at longest wavelength transition. ^{d} Fluorescence quantum yield in ethanol, determined using either 7-diethylamino-4-methylcoumarin (Φ_{F} = 0.73 in ethanol) as a standard. | | | | | |

### Theoretical calculations

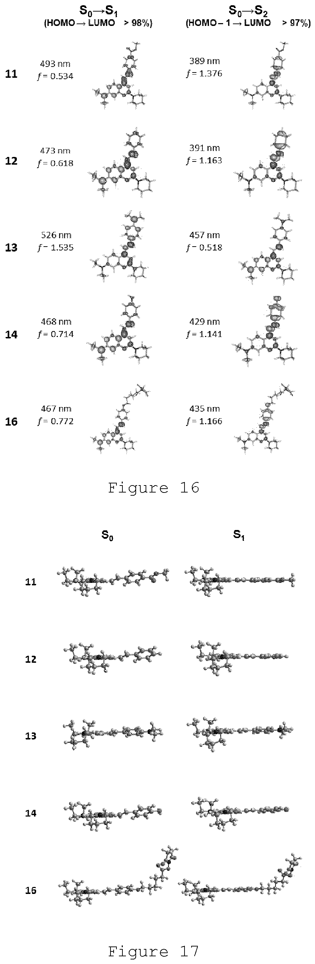

The experimental and calculated absorption spectra together with the frontier orbitals involved in the lowest energy transitions of a selection of coumarin derivatives are depicted in Figure 15. The general agreement between the calculated and the experimentally measured absorption spectra is quite good being able to capture it main features and to allow the assignment of the lowest energy absorption bands in terms of orbital transitions. For coumarin derivatives (11) and (12), the S0→S1 or S0→S2 transitions are well separated giving rise to the two absorption bands depicted in the experimental spectra. For the other compounds, these transitions present closer energies and coalesce to a single band as observed in the experimental spectra. Whereas in LUMO the charge is largely delocalized throughout the molecule, in the HOMO and HOMO-1 orbitals it is mainly localized either in the 4-styryl moiety or in the coumarin rings. However, while the HOMO state is mainly located on the coumarin moiety for compounds (11), (12), (14) and (16), for the compound (13) the HOMO state is mostly located in the 4-styryl group. The situation is the opposite for the HOMO-1 state which is located on the 4-styryl group for compounds (11), (12), (14) and (16) while for compound (13) is in the coumarin moiety. Comparing the absorption spectra and MOs of compounds (14) and (16) they are similar since the attached reactive group does not take part on the π-conjugation framework, thus justifying very similar photophysical properties. The maximum absorption wavelengths and the oscillator strength (f) of the key-transitions were calculated and are depicted in Figure 16 and Table 6, together with the assigned main components of the transitions. A visual picture of electronic rearrangement that occurs upon the lowest (and most important) transitions is given by electronic density differences of the states involved in the transitions. The electron density difference of the lowest energy excitations in displayed in Figure 16, illustrating the regions of the molecules that loose or gain electron charge. All compounds present two lowest-energy excitations S0→S1 and S0→S2 showing as final state the LUMO and as starting states the HOMO and HOMO-1 states, respectively. For all the compounds, excepting compound (13), S0→S1 accounts for an electron density flow from the 4-styryl group towards the coumarin moiety, whereas S0→S2 represents an electron density transfer to the 4-styryl group. For compound (13), however, the electron flows occur in the opposite direction. However, for all compounds, the most probable transition, i.e. that with the higher oscillator strength, despite being S0→S1 or S0→S2 corresponds for a decrease of electronic density in the 4-styryl pendent and a transfer of charge to the coumarin moiety. It is also for this compound that the higher oscillator strength corresponds to the lowest energy transition thus dominating the absorption spectrum and indicating the intense donor capability of the amine group bond to the 4-styryl moiety. A central factor that affects the emission process is the structural changes that possible occur on the geometry of the excited states after a vertical excitation [41, 42]. A large Stokes shift is expected if significant structural relaxation occurs in the excited state prior to emission. The optimized geometry of the studied coumarin derivatives in the electronic ground and first excited states are depicted in Figure 17. In the ground state the compounds show a significant deviation from planarity with the plane of 4-styryl group rotated relatively to the coumarin rings moiety plane. The torsion occurs on the bond that joins the coumarin rings moiety and the 4-styryl group, with the ethylene bridge being nearly in plane with the pendant benzyl group (see ESI for details). This dihedral angle present values between 15° for compound (13) and 26° for compound (11) and limits the π conjugation system that links the donor and acceptor groups in the molecules. On the excited S1 state however, after the structural relaxation, the dihedral angle changes to a minimum of 2° for compound (11) and a maximum of 7° for compound (13), therefore the molecules becoming more planar. Also, the calculated bond length alternation (BLA, the average difference between a single and adjacent double bond length) for the small 4-styryl conjugated chain, attains 0.11 A for compounds (11) and (12), 0.10 A for compound (14) and (16) and 0.08 A for compound (13), in the S0 state. For the S1 state the BLA reduces to 0.06 A for compound (13) and 0.04 A for all the other compounds. These changes in geometry contribute for an enhancement of the electronic delocalization throughout the π-conjugation framework and a decrease in the HOMO and LUMO energy gap, thus resulting in a smaller emission energy when compared with the excitation energy.

**Table 6. Calculated absorption data for the compounds and the main orbitals involved in the transitions.**

| Compound 11 | | | |
|---|---|---|---|
| State | λ (nm) | f | Major MO → MO transitions |
| S₁ | 494 | 0.534 | HOMO->LUMO (99%) |
| S₂ | 389 | 1.377 | H-1->LUMO (99%) |
| S₃ | 333 | 0.087 | H-2->LUMO (94%) |
| S₄ | 324 | 0.271 | HOMO->L+1 (94%) |
| S₅ | 304 | 0.041 | H-3->LUMO (93%) |

| Compound 12 | | | |
|---|---|---|---|
| State | λ (nm) | f | Major MO → MO transitions |
| S₁ | 473 | 0.618 | HOMO->LUMO (99%) |
| S₂ | 391 | 1.163 | H-1->LUMO (99%) |
| S₃ | 324 | 0.057 | H-2->LUMO (95%) |
| S₄ | 304 | 0.026 | H-3->LUMO (95%) |
| S₅ | 300 | 0.196 | HOMO->L+1 (89%) |
| | | | |

| Compound 13 | | | |
|---|---|---|---|
| State | λ (nm) | f | Major MO → MO transitions |
| S₁ | 526 | 1.535 | HOMO->LUMO (98%) |
| S₂ | 457 | 0.518 | H-1->LUMO (98%) |
| S₃ | 324 | 0.072 | H-3->LUMO (11%), H-2->LUMO (82%) |
| S₄ | 309 | 0.113 | H-3->LUMO (29%), HOMO->L+1 (56%) |
| S5 | 305 | 0.036 | H-3->LUMO (48%), H-2->LUMO (15%), H->L+1 (17%) |
| | | | |

| Compound 14 | | | |
|---|---|---|---|
| State | λ (nm) | f | Major MO → MO transitions |
| S₁ | 468 | 0.714 | HOMO->LUMO (98%) |
| S₂ | 429 | 1.141 | H-1->LUMO (98%) |
| S₃ | 322 | 0.047 | H-2->LUMO (93%) |
| S₄ | 296 | 0.150 | HOMO->L+1 (83%), HOMO->L+2 (10%) |
| S₅ | 292 | 0.012 | H-3->LUMO (85%) |
| | | | |

| Compound 16 | | | |
|---|---|---|---|
| State | λ (nm) | f | Major MO → MO transitions |
| S₁ | 467 | 0.772 | HOMO->LUMO (97%) |
| S₂ | 435 | 1.166 | H-1->LUMO (97%) |
| S₃ | 322 | 0.049 | H-2->LUMO (92%) |
| S₄ | 296 | 0.093 | H-3->LUMO (16%), HOMO->L+1 (68%) |
| S₅ | 294 | 0.051 | H-3->LUMO (66%), HOMO->L+1 (14%) |

### Materials and methods

### General methods

All starting materials and reagents were analytical grade, purchased from Aldrich and used without further purification. The organic solvents were dried over appropriate drying agents and distilled prior to use. UV-Vis absorption spectra were recorded on the Thermo Electron Spectrophotometer Corporation, model Nicolet Evolution 300, using acetonitrile (CH₃CN) as solvent. Fluorescence spectra were recorded on a PerkinElmer Model LS 55 spectrophotometer. FTMS-ESI mass spectra were obtained on an Thermo Scientific Q Exactive Orbitrap Mass Spectrometer. Nuclear magnetic resonance (NMR) spectra were recorded at 400MHz for ¹H NMR and 100MHz for ¹³C, on a Brucker Advance III spectrometer. For NMR spectra, deuterated chloroform (CDCl₃) or deuterated methanol (CD₃OD) was used as solvent. The chemical shift (δ) in ppm; coupling constants (J); relative intensity is indicated by the number of protons (H); multiplicities are indicated by singlet (s), doublet (d), double-doublet (dd), triplet (t), quadruple (q) and multiplet (m); coupling constants are given in Hertz (Hz).

### Procedures for the preparation of compounds 10 to 16

### 1-(7-(diethylamino)-4-methyl-2H-chromen-2-ylidene)-piperidin-1-ium nitrate (10)

A mixture of compound (2) (200 mg, 0.809 mmol, 1.0 eq), piperidine (120 µL, 1.21 mmol, 1.5 eq) and silver nitrate (343 mg, 2.02 mmol, 2.5 eq), in dry acetonitrile (10 mL) was stirred at room temperature for 2 hours. The reaction was continuously monitored by tlc, using CHCl₃/CH₃OH/H₂O (65:10:1). The reaction product was isolated by flash chromatography, using CHCl₃/CH₃OH (9:1) and CHCl₃/CH₃OH (8:2) as eluents, to yield 1-(7-(diethylamino)-4-methyl2H-chromen-2-ylidene)piperidin-1-ium nitrate (248 mg, 85%). ¹H NMR (400 MHz, CDCl₃): 1.24 (6H, t, J = 7.1 Hz, N(CH₂CH₃)₂),1.80-1.82 (6H, m, H-15, H-16, H-17), 2.58 (3H, s, 4-CH₃), 3.47 (4H, q, J = 7.1 Hz, N(CH₂CH₃)₂), 3.94-4.01 (4H, m, H-14, H-18), 6.61 (1H, d, J8,₆ = 2.4 Hz, H-8), 6.78 (1H, dd, J6,₅ = 9.2 Hz, J6,₈ = 2.4 Hz, H-6), 6.95 (1H, s, H-3), 7.56 (1H, d, J5,₆ = 9.2 Hz, H-5) .¹³C NMR (100 MHz, CDCl3): 12.5 (C-11, C-13), 19.4 (C-9), 23.6 (C-16), 26.0 (C-15, C-17), 45.2 (C-10, C-12), 46.8 (C14/C-18), 48.8 (C-14/C-18), 96.5 (C-8), 100.7 (C-3), 109.2 (C-4a), 111.8 (C-6), 127.0 (C-5), 158.4 (C-4), 152.6 (C-7), 154.2 (C-8a), 160.0 (C-2). FTMS (+) calc. for C₁₉H₂₇N₂O[M-(NO₃⁻)]⁺ 299.2117 found 299.2111. UV λ^{max} (nm, CH3CN): 261, 298, 437. ε (cm⁻¹ M⁻¹): 21000. ΦF = 0.14.

### (E)-1-(7-(diethylamino)-4-(4-(methoxycarbonyl)styryl)-2H-chromen-2ylidene)piperidin-1-ium nitrate (11)

A mixture of compound (10) (154 mg, 0.426 mmol, 1.0 eq), methyl 4-formylbenzoate (26 mg, 0.426 mmol, 1.0 eq) and piperidine (42 µL, 0.426 mmol, 1.0 eq), in dry acetonitrile (25 mL), was stirred at 85°C for 6 hours. The reaction was continuously monitored by tlc, using CHCl₃/CH₃OH (9:1) as eluent. The reaction product was isolate by flash chromatography, using CHCl₃ and CHCl₃/CH₃OH (8:2) as eluents, to yield (E)-1-(7-(diethylamino)-4-(4-(methoxycarbonyl)styryl)2H-chromen-2-ylidene)piperi-din-1-ium nitrate (130 mg, 60%). ¹H NMR (400 MHz, CDCl₃): 1.26 (6H, t, J = 7.2 Hz, N(CH₂CH₃)₂),1.80-1.89 (6H, m, H-15, H-16, H-17), 3.49 (4H, q, J = 7.2 Hz, N(CH₂CH₃)₂), 3.90 (3H, s, CH₃), 3.96-4.21 (4H, m, H-14, H-18), 6.53 (1H, d, J8,₆ = 2.4 Hz, H-8), 6.84 (1H, dd, J6,₅ = 9.2 Hz, J6,₈ = 2.4 Hz, H-6), 7.49 (1H, d, J19,₉ = 16 Hz, H-19), 7.52 (1H, s, H-3), 7.83 (2H, d, J21,₂₂ = 8.4 Hz, H-21, H-25), 7.89 (1H, d, J5,₆= 9.2 Hz, H-5), 7.95 (2H, d, J22,₂₁ = 8.4 Hz, H-22, H-24), 8.37 (1H, d, J9,₁₉ = 16 Hz, H-9).¹³C NMR (100 MHz, CDCl3): 12.6 (C-11, C-13), 23.8 (C-16), 26.2 (C15, C-17), 45.2 (C-10, C-12), 46.8 (C-14/C-18), 49.2 (C-14/C-18), 52.3 (OCH₃), 95.2 (C-3), 96.8 (C-8), 108.0 (C-4a), 112.0 (C-6), 120.8 (C-19), 127.0 (C-5), 128.6 (C-21,C-25), 129.9 (C-22, C-24), 130.8 (C23), 140.1 (C-4), 141.6 (C-9), 152.5 (C-7, C-20), 154.6 (C-8a), 160.0 (C-2), 166.7 (C-27). FTMS (+) calc. for C₂₈H₃₃O₃N₂[M-(NO₃⁻)]⁺ 445.24857 found 445.2479. UV λ^{max} (nm, CH₃CN): 362, 484. ε (cm⁻¹ M¹): 18000. ΦF = 0.08.

### (E)-1-(7-(diethylamino)-4-styryl-2H-chromen-2-ylidene)-piperidin-1-ium nitrate (12)

A mixture of compound (10) (276 mg, 0.764 mmol, 1.0 eq), benzaldehyde (93 µL, 0.764 mmol, 1.0 eq) and piperidine (76 µL, 0.755 mmol, 1.0 eq), in dry acetonitrile (10 mL) was stirred at 85 °C for 5 minutes. The reaction was continuously monitored by tlc, using CH₂Cl₂/hexane (8:2) as eluent. The reaction product was isolated by flash chromatography, using CH₂Cl₂/hexane (8:2) as eluent, to yield (E)-1-(7-(diethylamino)-4-styryl-2H-chromen-2-ylidene)piperidin-1-ium nitrate (309 mg, 90%). ¹H NMR (400 MHz, CDCl₃): 1.24 (6H, t, J = 7.2, N(CH₂CH₃)₂),1.73 -1.79 (6H, m, H-15, H-16, H-17), 3.46 (4H, q, J = 7.1 Hz, N(CH₂CH₃)₂), 3.91 - 3.97 (4H, m, H-14, H-18), 6.57 (1H, d, J8,₆ = 2.4 Hz, H-8), 6.81 (1H, dd, J6,₅ = 9.2 Hz, J6,₈ = 2.4, H-6), 7.18 (1H, s, H-3), 7.30-7.33 (3H, m, H-22, H-23, H-24), 7.35 (1H, d, J9,₁₉ = 16 Hz, H-9), 7.68 (2H, dd, J21,₂₂ = 8.0 Hz, J21,₂₃ = 2.0 Hz, H-21, H-25), 7.81 (1H, d, J5,₆ = 9.2 Hz, H-5), 8.01 (1H, d, J19,₉ = 16 Hz, H-19).¹³C NMR (100 MHz, CDCl₃): 12.6 (C-11, C-13), 23.7 (C-16), 26.1 (C-15, C-17), 45.2 (C-10, C-12), 46.8 (C-14/C-18), 48.7 (C-14/C-18), 93.9 (C3), 97.0 (C-8), 107.8 (C-4a),111.9 (C-6), 118.5 (C-9), 126.7 (C-5), 128.6 (C-21, C-25), 128.9 (C-22, C24), 130.2 (C-23), 135.6 (C-20), 142.5 (C-19), 152.5 (C-7), 152.9 (C-4), 154.6 (C-8a), 159.9 (C-2). FTMS(+) calc. for C₂₆H₃₁N₂O[M-(NO₃⁻)]⁺ 387.2431 found 387 . 2424 . UV λ^{max} (nm, CH3CN): 282, 301, 467. ε (cm⁻¹ M⁻¹): 17000. ΦF = 0.34.

### (E)-1-(7-(diethylamino)-4-(4-(dimethylamino)styryl)-2H-chromen-2ylidene)piperidin-1-ium nitrate (13)

A mixture of compound (10) (162 mg, 0.448 mmol, 1.0 eq), 4-(dimethylamino)benzaldehyde (67 mg, 0.448 mmol, 1.0 eq) and piperidine (44 µL, 0.445 mmol, 1.0 eq), in dry acetonitrile (10 mL) was stirred at 85 °C for 24 hours. The reaction was continuously monitored by tlc, using CHCl₃ as eluent. The reaction product was isolated by flash chromatography, using CH₂Cl₂/hexane (8:2), CH₂Cl₂/hexane (9:1) and CH₂Cl₂ as eluents, to yield (E)-1-(7-(diethylamino)-4-(4(dimethylamino)styryl)-2H-chromen-2-ylidene)piperidin-1-ium nitrate (205 mg, 92%). ¹H NMR (400 MHz, CDCl₃): 1.26 (6H, t, J = 7.2 Hz, N(CH₂CH₃)₂),1.80-1.82 (6H, m, H-15, H-16, H-17), 3.04 (6H, s, 2xNCH₃),3.49 (4H, q, J = 7.2 Hz N(CH₂CH₃)₂), 4.00 (4H, m, H-14, H-18), 6.59 (1H, d, J8,₆ = 2.4, H-8), 6.68 (2H, d, J = 8.8 Hz, H-22, H-24), 6.80 (1H, dd, J6,₅= 8.8 Hz, J6,₈ = 2.4 Hz, H-6), 7.10 (1H, s, H-3), 7.18 (1H, d, J 9,₁₇ = 15.8 Hz, H-9), 7.69 (1H, d, J = 8.8 Hz, H-21, H-25), 7.85 (1H, d, J5,₆= 8.8 Hz, H-5), 8.11 (1H, d, J 19,₉ = 15.8 Hz, H-19). ¹³C NMR (100 MHz, CDCl₃): 12.7 (C-11, C-13), 23.9 (C16), 26.2 (C-15, C-17), 40.3 (C-26, C-27), 45.2 (C-10, C-12), 47.5 (C-14/C-18), 48.4 (C-14/C-18), 91.5 (C-3), 97.0 (C-8), 108.2 (C-4a), 111.5 (C-6), 112.1 (C-22, C-24), 112.2 (C-9), 123.6 (C-20), 126.6 (C-5), 131.0 (C-21, C-25), 144.3 (C-19), 152.2 (C-7, C-23), 153.5 (C-4), 154.7 (C-8a), 159.8 (C-2). FTMS(+) calc. for C₂₈H₃₆N₃O[M-(NO₃⁻)]⁺ 430 . 2853 found 430.2845. UV λ^{max} (nm, CH3CN): 282, 485. ε (cm⁻¹ M⁻¹): 39000. ΦF = 0.98.

### (E)-1-(7-(diethylamino)-4-(4-methoxystyryl)-2H-chromen-2-ylidene)piperidin-1-ium nitrate (14)

A mixture of compound (10) (150 mg, 0.415 mmol, 1.0 eq), 4-methoxybenzaldehyde (50 µL, 0.415 mmol, 1.0 eq) and piperidine (41 µL, 0.415 mmol, 1.0 eq) in dry acetonitrile (10 mL), was stirred at 85 °C for 18 hours. The reaction was continuously monitored by tlc, using CHCl₃/CH₃OH/H₂O (65:20:2) as eluent. The reaction product was isolated by flash chromatography, using CHCl₃/CH₃OH/H₂O (65:20:2) as eluent, to yield (E)-1-(7-(diethylamino)-4-(4-methoxystyryl)-2Hchromen-2-ylidene)piperidin-1-ium nitrate (171 mg, 86%). ¹H RMN (400 MHz, CDCl₃): 1.24 (6H, t, J = 7.1, N(CH₂CH₃)₂), 1.75-1.80 (6H, m, H-15, H-16, H-17), 3.47 (4H, q, J = 7.1, N(CH₂CH₃)₂), 3.79 (3H, s, H-26), 3.97 (4H, m, H-14, H-18), 6.57 (1H, d, J8,₆ = 2.2 Hz, H-8), 6.79 (1H, dd, J6,₅ = 9.2 Hz, J6,₈ = 2.2 Hz, H-6), 6.85 (2H, d, J22,₂₁ = 8.6 Hz, H-22, H-24), 7.18 (1H, s, H-3), 7.24 (1H, d, J9,₁₉ = 15.9 Hz, H-9), 7.70 (1H, d, J21,₂₂ = 8.6 Hz, H-21, H-25), 7.84 (1H, d, J5,₆= 9.2 Hz, H-5), 8.06 (1H, d, J 19,₉ = 15.8 Hz, H-19).¹³C NMR (100 MHz, CDCl₃): 12.6 (C-11, C-13), 23.9 (C-16), 26.1 (C-15, C-17), 44.6 (C14/C-18) 45.2 (C-10, C-12), 46.5 (C-14/C-18), 55.5 (C-26), 93.0 (C-3), 97.0 (C-8), 108.0 (C-4a), 111.7 (C-6), 114.4 (C-22, C-24), 115.8 (C-9), 126.7 (C-5), 128.5 (C-20), 131.6 (C-21, C-25), 142.7 (C-19), 152.4 (C-7), 153.2 (C-4), 154.7 (C-8a), 159.9 (C-2), 161.6 (C-23). FTMS(+) calc. for C₂₇H₃₃O₂N₂[M-(NO₃⁻)]⁺ 417.25365 found 417.2527. UV λ^{max} (nm, CH3CN): 410. ε (cm⁻¹ M⁻¹): 30000. ΦF = 0.82.

### (E)-1-(4-(4-((5-carboxypentyl)oxy)styryl)-7-(diethylamino)-2H-chromen-2ylidene)piperidin-1-ium nitrate (15)

A mixture of compound (10) (232 mg, 0.642 mmol, 1.0 eq), 6-(4-formylphenoxy) hexanoic acid (152 mg, 0.642 mmol, 1.0 eq) and piperidine (63 µL, 0.642 mmol, 1.0 eq) in dry acetonitrile (10 mL) was stirred at 85°C for 5h. The reaction was continuously monitored by tlc using CHCl₃/CH₃OH (9:1) as eluent. The reaction product was isolated by flash chromatography, using CHCl₃/CH₃OH (95:5), CHCl₃/CH₃OH (9:1) and CHCl₃/CH₃OH (8:2) as eluents, to yield (E)-1-(4-(4-((5carboxypentyl)oxy)styryl)-7-(diethylamino)-2H-chromen-2-ylidene)piperidin-1-ium nitrate (261 mg, 70%). ¹H RMN (400 MHz, CD₃OD): 1.26 (6H, t, J = 7.1 Hz, N(CH₂CH₃)₂), 1.53 (2H, m, H-28), 1.69 (2H, m, H-29), 1.78-1.83 (2H, m, H-27), 1.83 (6H, m, H-15, H-16, H-17), 2.32 (2H, t, H-30), 3.56 (4H, q, J = 7.1 Hz, N(CH₂CH₃)₂), 3.97 (4H, m, H-14, H-18), 4.02 (2H, t, H-26), 6.84 (1H, d, J8,₆ = 2.2 Hz, H-8), 6.95-6.98 (1H, dd, J6,₅ = 9.2 Hz, J6,₈ = 2.2 Hz, H-6), 6.98 (1H, s, H-3), 6.96 (2H, d, J = 8.4 Hz, H-22, H-24), 7.49 (1H, d, J9,₁₉= 15.9 Hz, H-9), 7.70 (2H, d, J = 8.4 Hz, H-21, H-25), 7.83 (1H, d, J19,₉= 15.9 Hz, H-19), 8.03 (1H, d, J5,₆ = 9.2 Hz, H-5) . ¹³C NMR (100 MHz, CD₃OD): 12.8 (C-11, C-13), 24.8 (C-16), 26.1 (C-29), 26.8 (C-28), 27.0 (C-15, C-17), 30.0 (C-27), 35.6 (C-30), 45.7 (C-14/C-18), 46.0 (C10, C-12), 47.6 (C-14/C-18), 69.1 (C-26), 93.0 (C-3), 98.0 (C-8), 109.0 (C-4a), 113.0 (C-6), 116.1 (C-22, C-24), 117.5 (C-9), 127.9 (C-5), 129.6 (C-20), 131.3 (C-21, C-25), 142.5 (C-19), 154.1 (C-7), 154.7 (C4), 156.3 (C-8a), 161.5.9 (C-2), 162.7 (C-23). FTMS (+) calc. for C₃₂H₄₁O₄N₂[M-(NO₃⁻)]⁺ 517.30608 found 517.3055. UV λ^{max} (nm, CH3CN): 406, 478. ε (cm⁻¹ M⁻¹): 30000. ΦF = 0.90.

### (E)-1-(7-(diethylamino)-4-(4-((6-((2,5-dioxopyrrolidin-1-yl)oxy)-6oxohexyl)oxy)styryl)-2H-chromen-2-ylidene)piperidin-1-ium nitrate (16)

A mixture of compound (15) (160 mg, 0.309 mmol, 1.0 eq), DCC (77 mg, 0.371 mmol, 1.2 eq), DMAP (4 mg, 0.0309 mmol, 10%) in dry DMF (2 mL) and dry acetonitrile (25 mL), was stirred at room temperature for 5 minutes. After this period N-hydroxysuccinimide (43 mg, 0.371 mmol, 1.2 eq) was added and the reaction mixture was stirred at room temperature for 6h. The reaction was monitored by tlc, using CHCl₃/CH₃OH/H₂O (65:20:2) as eluent. The reaction product was isolated by flash chromatography, using CHCl₃/CH₃OH (95:5), CHCl₃/CH₃OH (9:1) and CHCl₃/CH₃OH (8:2) as eluents, to yield (E)-1-(7-(diethylamino)-4-(4-((6-((2,5-dioxopyrrolidin-1-yl)oxy)-6oxohexyl)oxy)styryl)-2H-chromen-2-ylidene)piperidin-1-ium nitrate (200 mg, 98%). ¹H RMN (400 MHz, CDCl₃): 1.22 (6H, t, J = 7.1 Hz, N(CH2CH3)2), 1.57 (2H, m, H-28), 1.72-1.82 (2H, m, H15, H-16, H-17, H-27, H-29), 2.61 (2H, t, H-30), 2.82 (4H, s, H-33, H-34), 3.45 (4H, q, J = 7.1 Hz, N(CH₂CH₃)₂), 3.92 (4H, m, H-14, H-18), 3.93 (2H, t, H-26), 6.55 (1H, d, J8,₆ = 2.3 Hz, H-8), 6.79 (1H, dd, J6,₅ = 9.2 Hz, J6,₈ = 2.3 Hz, H-6), 6.82 (2H, d, J = 8.8 Hz, H-22, H-24), 7.07 (1H, s, H-3), 7.22 (1H, d, J9,₁₉= 15.9 Hz, H-9), 7.64 (2H, d, J = 8.8 Hz, H-21, H-25), 8.82 (1H, d, J5,₆= 9.2 Hz, H-5), 7.94 (1H, d, J19,₉= 15.9 Hz, H-19).¹³C NMR (100 MHz, CDCl₃): 12.6 (C-11, C-13), 23.7 (C-16), 24.4 (C-29), 25.3 (C-28), 25.7 (C-33, C-34), 26.0 (C-15, C-17), 28.7 (C-27), 31.0 (C-30), 45.1 (C-10, C-12), 46.7 (C-14/C18), 48.4 (C-14/C-18), 67.7 (C-26), 92.7 (C-3), 96.9 (C-8), 107.9 (C-4a), 111.8 (C-6), 115.0 (C-22, C-24), 115.7 (C-9), 126.7 (C-5), 128.3 (C-20), 130.5 (C-21, C-25), 142.5 (C-19), 152.4 (C-7), 153.1 (C-4), 154.6 (C-8a), 159.8 (C-2), 161.0 (C-23), 168.6 (C-31), 169.4 (C-32, C-35). FTMS (+) calc. for C₃₆H₄₄O₆N₃[M(NO₃⁻)]⁺ 614.32246 found 614.3215. UV λ^{max} (nm, CH3CN): 404, 480. ε (cm⁻¹ M⁻¹): 30000. ΦF = 0.92.

### Quantum chemical calculations

All quantum chemical calculations were carried out with the Gaussian 16 software package [44] . The hybrid PBE0 functional [45] together with the standard 6-31G(d,p) basis set was used for geometry optimizations of both the ground and first excited-states, without any symmetry constraints, while the larger 6-311+G(d,p) basis set was employed for the spectra calculations. Solvent effects were treated by means of the implicit polarized continuum model (PCM) [47, 48]. Frequency analysis was performed both in the ground and excited stare, confirming the optimized structures as minima, presenting all frequencies real valued.

Table 7 shows the calculated emission data for the compounds.

**Table7. Calculated emission data for the studied compounds and the main orbitals involved in the S₁→S₀ transitions.**

| Compound | λ (nm) | f | Major MO → MO transitions |
|---|---|---|---|
| 11 | 578 | 0.450 | HOMO->LUMO (99%) |
| 12 | 539 | 0.530 | HOMO->LUMO (99%) |
| 13 | 558 | 1.655 | HOMO->LUMO (99%) |
| 14 | 525 | 0.647 | HOMO->LUMO (99%) |
| 16 | 523 | 0.702 | HOMO->LUMO (99%) |

### Bibliography

1. Modesti, M. Fluorescent Labeling of Proteins. Methods Mol. Biol. 2018, 1665, 115-134.
2. Nath, N.; Becky Godat, B.; Urh, M. Antibody Labeling with Fluorescent Dyes Using Magnetic Protein A and Protein G Beads. J. Vis Exp. 2016, 115, e54545.
3. Dirks, R.W.; Tanke, H.J. Advances in fluorescent tracking of nucleic acids in living cell. Biotechniques 2006, 40, 489-495.
4. Rogera, O.; Colliec-Jouaulta, S.; Ratiskola, J.; Sinquina, C.; Guezenneca, J.; Fischerb, A.M.; Chevolota, L. Polysaccharide labelling: Impact on structural and biological properties. Carbohyd. Polym. 2002, 50, 273-278.
5. Maekawa, M.; Fairn, G.D. Molecular probes to visualize the location, organization and dynamics of lipids. J. Cell Sci. 2014, 127, 4801-4812.
6. Pereira, A.; Martins, S.; Caldeira, A.T. Coumarins as Fluorescent Labels of Biomolecules. In Phytochemicals in Human Health; Rao, V., Ed.; IntechOpen Publications: London, UK, 2019, pp. 1-19.
7. Giepmans, B.; Adams, S.; Ellisman, M.; Tsien, R. The fluorescent toolbox for assessing protein location and function. Science 2006, 312, 217-224.
8. Zimmer, M. Green fluorescent protein (GFP): Applications, structure, and related photophysical behavior. Chem. Rev. 2002, 102, 759-781.
9. Staveren, D.; Metzler-Nolte, N. Bioorganometallic Chemistry of Ferrocene. Chem. Rev. 2004, 104, 5931-5985.
10. Soh, N.; Yoshida, K.; Nakajima, H.; Nakano, K.; Imato, T.; Fukaminato, T.; Irie, M. A fluorescent photochromic compound for labeling biomolecules. Chem. Commun. 2007, 48, 5206-5208.
11. Benedetto, F.; Mele, E.; Camposeo, A.; Athanassiou, A.; Cingolani, R.; Pisignano, D. Photoswitchable Organic Nanofibers. Adv. Mater. 2008, 20, 314-318.
12. Li, H.; Rothberg, L. Colorimetric detection of DNA sequences based on electrostatic interactions with unmodified gold nanoparticles. Proc. Natl. Acad. Sci. USA 2004, 101, 14036-14039.
13. Varki, A. Radioactive tracer techniques in the sequencing of glycoprotein oligosaccharides. FASEB J. 1991, 5, 226-235.
14. Ong, S.; Mann, M. A practical recipe for stable isotope labeling by amino acids in cell culture (SILAC). Nat. Protoc. 2006, 1, 2650-2660.
15. Sahoo, H. Fluorescent labeling techniques in biomolecules: A flashback. RSC Adv. 2012, 2, 7017-7029.
16. Hanson, G.; Hanson, B. Fluorescent probes for cellular assays. Comb. Chem. High Throughput Screen. 2008, 11, 505-513.
17. Maurel, D.; Comps-Agrar, L.; Brock, C.; Rives, M.; Bourrier, E.; Ayoub, M.; Bazin, H.; Tinel, N.; Durroux, T.; Prezeau, L.; et al. Cell-surface protein-protein interaction analysis with time-resolved FRET and snap-tag technologies: Application toGPCR oligomerization. Nat. Methods 2008, 5, 561-567.
18. Yin, J.; Straight, P.; McLoughlin, S.; Zhou, Z.; Lin, A.; Golan, D.; Kelleher, N.; Kolter, R.; Walsh, C. Genetically encoded short peptide tag for versatile protein labeling by Sfp phosphopantetheinyl transferase. Proc. Natl. Acad. Sci. USA 2005, 102, 15815-15820.
19. Hermanson, T. Bioconjugate Techniques, 3rd ed.; Elsevier: London, UK, 2013.
20. Fang, X.; Zheng, Y.; Duan, Y.; Liu, Y.; Zhong, W. Recent Advances in Design of Fluorescence-Based Assays for High-Throughput Screening. Anal. Chem. 2019, 91, 482-504.
21. Johnson, I.; Spence, M. Molecular Probes™ Handbook-A Guide to Fluorescent Probes and Labeling Technologies, 11th ed.; Life Technologies; Thermo Fischer Scientific: Waltham, MA, USA, 2010.
22. Kobayashi, H.; Ogawa, M.; Alford, R.; Choyke, P.; Urano, Y. New Strategies for Fluorescent Probe Design in Medical Diagnostic Imaging. Chem. Rev. 2009, 110, 2620-2640.
23. Barot, K.P.; Jain, S.V.; Kremer, L.; Singh, S.; Ghate, M.D. Recent advances and therapeutic journey of coumarins: Current status and perspectives. Med. Chem. Res. 2015, 24, 2771-2798.
24. Riveiro, M.; De Kimpe, N.; Moglioni, A.; Vázquez, R.; Monczor, F.; Shayo, C.; Davio, C. Coumarins: Old compounds with novel promising therapeutic perspectives. Curr. Med. Chem. 2010, 17, 1325-1338.
25. Lee, M.; Yen, C.; Yang, W.; Chen, H.; Liao, C.; Tsai, C.; Chen, C. Efficient green coumarin dopants for organic light-emitting devices. Org. Lett. 2004, 6, 1241-1244.
26. Martins, S.; Avó, J.; Lima, J.; Nogueira, J.; Andrade, L.; Mendes, A.; Pereira, A.; Branco, P.S. Styryl and phenylethynyl based coumarin chromophores for dye sensitized solar cells. J. Photochem. Photobiol. A Chem. 2018, 353, 564-569.
27. Lanke, S.K.; Sekar, N. Coumarin Push-Pull NLOphores with red emission: Solvatochromic and theoretical approach. J. Fluoresc. 2016, 26, 949-962.
28. Zhang, G.; Zheng, H.; Guo, M.; Du, L.; Liu, G.; Wang, P. Synthesis of polymeric fluorescent brightener based on coumarin and its performances on paper as light stabilizer, fluorescent brightener and surface sizing agent. Appl. Surf. Sci. 2016, 367, 167-173.
29. Wilze, K.; Johnson, A. Handbook of Detergents, Chemistry, Production, and Application of Fluorescent Whitening Agents, Part, F; Taylor & Francis. CRC Press: Boca Raton, FL, USA, 2007.
30. Zaorska, E.; Konop, M.; Ostaszewski, R.; Koszelewski, D.; Ufnal, M. Salivary Hydrogen Sulfide Measured with a New Highly Sensitive Self-Immolative Coumarin-Based Fluorescent Probe. Molecules 2018, 23, 2241.
31. Gandioso, A.; Bresoli-Obach, R.; Nin-Hill, A.; Bosch, M.; Palau, M.; Galindo, A.; Contreras, S.; Rovira, A.; Rovira, C.; Nonell, S.; et al. Redesigning the coumarin scaffold into small bright fluorophores with far-red to NIR emission and large Stokes' shifts useful for cell imaging. J. Org. Chem. 2018, 83, 1185-1195.
32. Bojtár, M.; Kormos, A.; Kis-Petik, K.; Kellermayer, M.; Kele, P. Green-Light Activatable, Water-Soluble Red-Shifted Couma-rin Photocages. Org. Lett. 2019, 21, 9410-9414.
33. Martins, S.; Branco, P.; Pereira, A. An Efficient Methodology for the Synthesis of 3-Styryl Coumarins. J. Braz. Chem. Soc. 2012, 23, 688-693.
34. Gordo, J.; Avó, J.; Parola, J.; Lima, J.; Pereira, A.; Branco, P. Convenient Synthesis of 3-Vinyl and 3-Styryl Coumarins. Org. Lett. 2011, 13, 5112-5115.
35. Gandioso, A.; Contreras, S.; Melnyk, I.; Oliva, J.; Nonell, S.; Velasco, D.; Garcia-Amorós, J.; Marchán, V. Development of green/red-absorbing chromophores based on a coumarin scaffold useful as caging groups. J. Org. Chem. 2017, 82, 5398-5408.
36. Joo, J.; Won, M.; Park, S.; Park, K.; Shin, D.-S.; Kim, J.; Lee, M. A dicyanocoumarin-fused quinolinium based probe for NAD(P)H and its use for detecting glycolysis and hypoxia in living cells and tumor spheroids. Sens. Actuators B Chem. 2020, 320, 128360.
37. Cao, D.; Liu, Z.; Verwilst, P.; Koo, S.; Jangjili, P.; Kim, J.S.; Lin, W. Coumarin-Based Small-Molecule Fluorescent Chemosensors. Chem. Rev. 2019, 119, 10403-10519.
38. Fu, Y.; Finney, N.S. Small-molecule fluorescent probes and their design. RSC Adv. 2018, 8, 29051-29061.
39. Sednev, M.V.; Belov, V.N.; Hell, S.W. Fluorescent dyes with large Stokes shifts for super-resolution optical microscopy of biological objects: A review. Methods Appl. Fluoresc. 2015, 3, 042004.
40. Cisse, L.; Djande, A.; Capo-Chichi, M.; Khonté, A.; Bakhoum, J.; Delattre, F.; Yoda, J.; Saba, A.; Tine, A.; Aaron, J. Quantitative study of the substituent effects on the electronic absorption and fluorescence spectra of coumarins. J. Phys. Org. Chem. 2019, 33, e4014.
41. Chen, Y.; Zhao, J.; Guo, H.; Xie, L. Geometry Relaxation-Induced Large Stokes Shift in Red-Emitting Borondipyrromethenes (BODIPY) and Applications in Fluorescent Thiol Probes. J. Org. Chem. 2012, 77, 2192-2206.
42. Ma, J.; Zhang, Y.; Zhang, H.; He, X. Near infrared absorption/emission perylenebisimide fluorophores with geometry relaxation-induced large Stokes shift. RSC Adv. 2020, 10, 35840-35847.
43. Jaggi, M.; Blum, C.; Marti, B.S.; Liu, S.X.; Leutwyler, S.; Decurtins, S. Annulation of Tetrathiafulvalene to the Bay Region of Perylenediimide. Org. Lett. 2010, 12, 1344-1347.
44. Gaussian 16, Revision B.01, Frisch, M. J., Trucks, G. W., Schlegel, H. B., Scuseria, G. E., Robb, M. A., Cheeseman, J. R.; Scalmani, G.; Barone, V.; Petersson, G. A.; Nakatsuji, H.; Li, X.; Caricato, M.; Marenich, A. V.; Bloino, J., Janesko, B. G., Gomperts, R., Mennucci, B., Hratchian, H. P., Ortiz, J. V., Izmaylov, A. F., Sonnenberg, J. L., Williams-Young, D., Ding, F., Lipparini, F., Egidi, F., Goings, J., Peng, B., Petrone, A., Henderson, T., Ranasinghe, D., Zakrzewski, V. G., Gao, J., Rega, N., Zheng, G., Liang, W., Hada, M., Ehara, M., Toyota, K., Fukuda, R., Hasegawa, J., Ishida, M., Nakajima, T., Honda, Y., Kitao, O., Nakai, H., Vreven, T., Throssell, K., Montgomery Jr., J. A., Peralta, J. E., Ogliaro, F., Bearpark, M. J., Heyd, J. J., Brothers, E. N., Kudin, K. N., Staroverov, V. N., Keith, T. A., Kobayashi, R., Normand, J., Raghavachari, K., Rendell, A. P., Burant, J. C., Iyengar, S. S., Tomasi, J., Cossi, M., Millam, J. M., Klene, M., Adamo, C., Cammi, R., Ochterski, J. W., Martin, R. L., Morokuma, K., Farkas, O., Foresman, J. B., Fox, D. J. Gaussian, Inc., Wallingford CT, 2016.
45. Adamo, C.; Barone, V. Toward reliable density functional methods without adjustable parameters: The PBE0 model. J. Chem. Phys. 1999, 110, 6158-6170.
46. Wang, J.; Durbeej, B. How accurate are TD-DFT excited-state geometries compared to DFT ground-state geometries? J. Comput. Chem. 2020, 41, 1718-1729.
47. Amovilli, C.; Barone, V.; Cammi, R.; Cancès, E.; Cossi, M.; Mennucci, B.; Pomelli, C. S.; Tomasi, J. Recent Advances in the Description of Solvent Effects with the Polarizable Continuum Model. J. Adv. Quantum Chem. 1998, 32, 227-261.
48. Cossi, M.; Barone, V. Time-dependent density functional theory for molecules in liquid solutions. J. Chem. Phys. 2001, 115, 4708-4717.
49. Xiao, Y.; Qian, X. Substitution of oxygen with silicon: A big step forward for fluorescent dyes in life science. Coord. Chem. Rev. 2020, 423, 213513.

## Claims

1. Fluorescent 4-styrylcoumarin derivative compounds of Formula I and Formula II: 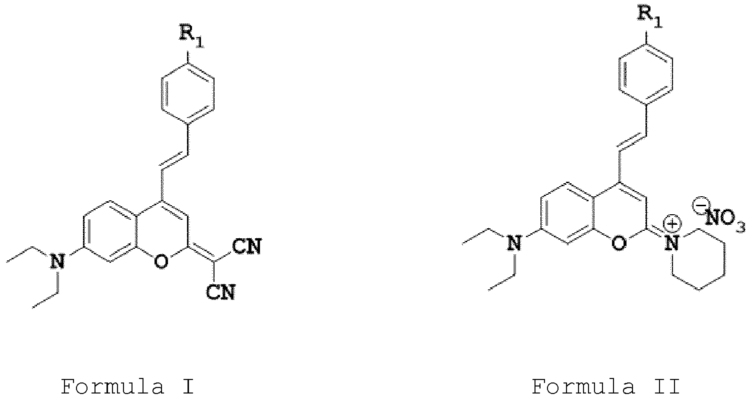 wherein
R₁ is selected from CO₂CH₃, H, N(CH₃)₂, OCH₃, 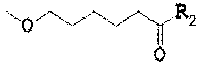 or 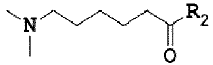
and R₂ is selected from OH,

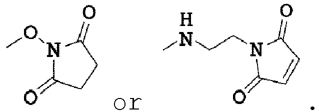

2. Fluorescent 4-styrylcoumarin derivative compounds of Formula III: 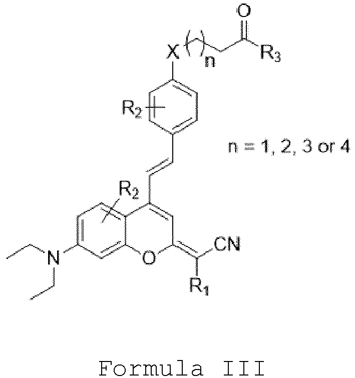 wherein
X is selected from NCH₃, O, N or S;
R₁ is selected from:
-COOCH₃, -COC₆H₄COOCH₃, -C₆H₄COOCH₃, -CN or 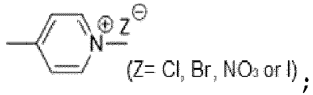
R₂ is selected from H or SO₃Na;
R₃ is selected from:
OH,

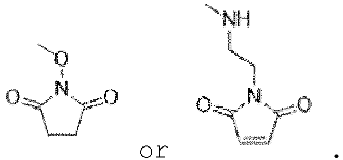

3. Fluorescent 4-styrylcoumarin derivative compounds of Formula IV: 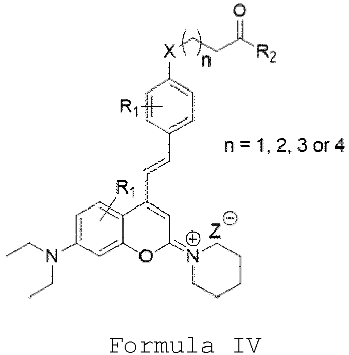 wherein
X is selected from NCH₃, O, N or S;
Z is selected from Cl, Br, NO₃ or I;
R₁ is selected from H or SO₃Na;
R₂ is selected from:
OH,

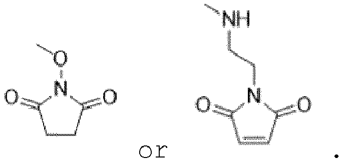

4. Fluorescent 4-styrylcoumarin derivative compounds of Formula I, Formula II, Formula III or Formula IV according to claim 1, 2 or 3, wherein the compounds are (E)-6-(4-(2-(2-(dicyanomethylene)-7-(diethylamino)-2H-chromen-4yl)vinyl)phenoxy)hexanoic acid (8); (E)-2,5-dioxopyrrolidin-1-yl 6-(4-(2-(2-(dicyanomethylene)-7-(diethylamino)-2H-chromen-4-yl)vinyl)phenoxy)hexanoate (9); (E)-1-(4-(4-((5-carboxypentyl)oxy)styryl)-7-(diethylamino)-2H-chromen-2ylidene)piperidin-1-ium nitrate (15); (E)-1-(7-(diethylamino)-4-(4-((6-((2,5-dioxopyrrolidin-1-yl)oxy)-6oxohexyl)oxy)styryl)-2H-chromen-2-ylidene)piperidin-1-ium nitrate (16).

5. Fluorescent 4-styrylcoumarin derivative compounds of Formula I and II according to claim 1 for use as labels for biomolecules.

6. Fluorescent 4-styrylcoumarin derivative compounds of Formula III according to claim 2 for use as labels for biomolecules.

7. Fluorescent 4-styrylcoumarin derivative compounds of Formula IV according to claim 3 for use as labels for biomolecules.
